# EUROPÄISCHE PATENTSCHRIFT

(11) **EP 1 465 870 B1**
(45) Veröffentlichungstag und Bekanntmachung des Hinweises auf die Patenterteilung: **03.05.2006**
(21) Anmeldenummer: 02795126.8
(22) Anmeldetag: 09.12.2002
(51) Int. Cl.: C07D 217/10, C07D 217/14, A61K 31/472, A61P 9/06

(54) **SUBSTITUIERTE 4-PHENYLTETRAHYDROISOCHINOLINIUM-SALZE, VERFAHREN ZU IHRER HERSTELLUNG, IHRER VERWENDUNG ALS MEDIKAMENT, SOWIE SIE ENTHALTENDES MEDIKAMENT**
SUBSTITUTED 4-PHENYLTETRAHYDROISOQUINOLINIUM SALTS, METHOD FOR PRODUCTION AND USE THEREOF AS A MEDICAMENT AND MEDICAMENTS COMPRISING THE SAME
SELS DE 4-PHENYLTETRAHYDROISOCHINOLINIUM SUBSTITUES, PROCEDE DE FABRICATION, UTILISATION EN TANT QUE MEDICAMENT, ET MEDICAMENT CONTENANT CES SELS

(30) Priorität: 22.12.2001 DE 10163914
(43) Veröffentlichungstag der Anmeldung: 13.10.2004
(73) Patentinhaber: Sanofi-Aventis Deutschland GmbH, 65929 Frankfurt am Main (DE)
(72) Erfinder: HOFMEISTER, Armin, 55276 Oppenheim (DE); LANG, Hans-Jochen, 65719 Hofheim (DE); HEINELT, Uwe, 65187 Wiesbaden (DE); BLEICH, Markus, 65597 Hünfelden-Dauborn (DE); WIRTH, Klaus, 65830 Kriftel (DE)
(86) Internationale Anmeldenummer: PCT/EP2002/013922
(87) Internationale Veröffentlichungsnummer: WO 2003/055880

(56) Entgegenhaltungen:
- EP-A- 0 659 748
- DE-A- 10 019 062
- PENELOPE A. DANDRIDGE ET AL.: "Synthesis, resolution, absolute stereochemistry, and enantioselectivity of 3',4'-dihydroxynomifensine" JOURNAL OF MEDICINAL CHEMISTRY., Bd. 27, Nr. 1, - 1984 Seiten 28-35, XP000567038 AMERICAN CHEMICAL SOCIETY. WASHINGTON., US ISSN: 0022-2623
- MASARU KIHARA ET AL.: "Resolution, absolute stereochemistry, and enantioselectivity of 2-methyl-4-phenyl-1,2,3,4-tetrahydroisoqui nolin-4-ol" JOURNAL OF MEDICINAL CHEMISTRY., Bd. 33, Nr. 8, - 1990 Seiten 2283-2286, XP002250030 AMERICAN CHEMICAL SOCIETY. WASHINGTON., US ISSN: 0022-2623
- CHEMICAL ABSTRACTS, vol. 87, no. 13, 26. September 1977 (1977-09-26) Columbus, Ohio, US; abstract no. 102137p, EIDEN, FRITZ ET AL.: "Analysis of psychopharmaceuticals: nomifensine (Alival)." XP002250031 & DTSCH. APOTH.-ZTG., Bd. 117, Nr. 17, - 1977 Seiten 611-614, -& DATABASE CAPLUS [Online] CHEMICAL ABSTRACTS SERVICE, COLUMBUS, OHIO, US; Database accession no. 87:102137 XP002250032

## Beschreibung

Die Erfindung betrifft Verbindungen der Formel I 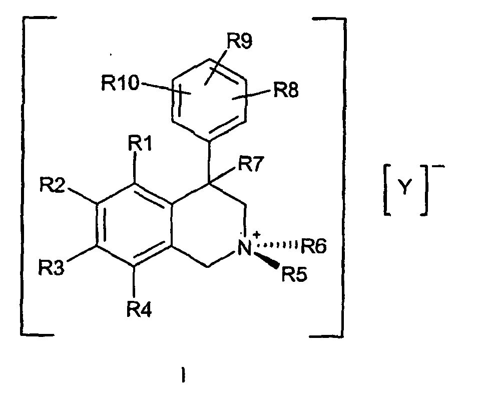 worin bedeuten:
- R1, R2, R3 und R4: unabhängig voneinander H, F, Cl, Br, I, CN, NO₂, OH, NH₂, CₐH₂ₐ₊₁, C_{qq}H_{2qq-1}, OC_{b}H_{2b+1}, COOR50, OCOR50, COR50 oder Oₓ-(CH₂)_{y}-Phenyl;
a und b in den Gruppen CₐH₂ₐ₊₁, und OC_{b}H_{2b+1} unabhängig voneinander 1, 2, 3, 4, 5, 6, 7 oder 8, wobei ein oder mehrere H-Atome durch F-Atome ersetzt sein können;
qq 3, 4, 5,6, 7 oder 8, wobei ein oder mehrere H-Atome durch F-Atome ersetzt sein können;
R50 H oder C_{c}H_{2c+1};
c 1, 2, 3, 4, 5, 6, 7 oder 8, wobei ein oder mehrere H-Atome durch F-Atome ersetzt sein dürfen,
x Null oder 1;
y Null, 1, 2, 3 oder 4;
wobei der Phenylring in der Gruppe Oₓ-(CH₂)_{y}-Phenyl unsubstituiert ist oder substituiert mit 1 - 3 Substituenten ausgewählt aus der Gruppe bestehend aus F, Cl, Br, CN, NO₂, OH, NH₂ oder C_{d}H_{2d+1};
d 1, 2, 3, oder 4, wobei ein oder mehrere H-Atome durch F-Atome ersetzt sein dürfen;
oder
- R1, R2, R3 und R4: unabhängig voneinander Heteroaryl, wobei Null, 1, 2, 3 oder 4 N-Atome, Null oder 1 Sauerstoff-Atom oder Null oder 1 S-Atom als Ringatome enthalten sein können;
oder
- R1, R2, R3 und R4: unabhängig voneinander CONR11R12 oder NR11R12;
R11 und R12 unabhängig voneinander H, CₑH₂ₑ₊₁, CᵣᵣH₂ᵣᵣ₋₁;
e 1, 2, 3, 4, 5, 6, 7 oder 8,
rr 3, 4, 5, 6, 7, oder 8,
wobei in den Gruppen CₑH₂ₑ₊₁ und CᵣᵣH₂ᵣᵣ₋₁ ein oder mehrere H-Atome durch F-Atome ersetzt sein dürfen und ein oder mehrere CH₂-Gruppen durch O oder NR13 ersetzt sein können;
R13 H oder C_{f}H_{2f+1};
f 1, 2, 3, oder 4, wobei ein oder mehrere H-Atome durch F-Atome ersetzt sein dürfen; oder
R13 und eine CH₂-Gruppe von R11 oder R12 gemeinsam mit dem N-Atom, an das sie gebunden sind, einen 5- oder 6-gliedrigen Ring; oder
R11 und R12 gemeinsam mit dem N-Atom, an das sie gebunden sind, einen 5-, 6- oder 7-gliedrigen Ring;
oder
R11 und R12 unabhängig voneinander COR14, CSR14 oder SO₂R14;
R14 C_{g}H_{2g+1};
g 1, 2, 3, 4, 5, 6, 7 oder 8, wobei ein oder mehrere H-Atome durch F-Atome ersetzt sein können und ein oder mehrere CH₂-Gruppen durch O oder NR13 ersetzt sein können,
oder
- R1, R2, R3 und R4: unabhängig voneinander -Oₕ-SOⱼ-R15, wobei
h Null oder 1;
j Null, 1 oder 2;
R15 CₖH₂ₖ₊₁, OH, OCₗH₂ₗ₊₁ oder NR17R18;
k 1, 2, 3, 4, 5, 6, 7 oder 8, wobei ein oder mehrere H-Atome durch F-Atome ersetzt sein können;
l 1, 2, 3, 4, 5, 6,7 oder 8, wobei ein oder mehrere H-Atome durch F-Atome ersetzt sein können;
R17 und R18 unabhängig voneinander H oder CₘH₂ₘ₊₁;
m 1, 2, 3, 4, 5, 6, 7 oder 8, wobei in der Gruppe CₘH₂ₘ₊₁ ein oder mehrere H-Atome durch F-Atome ersetzt sein können und ein oder mehrere CH₂₋Gruppen durch O, CO, CS oder NR19 ersetzt sein können;
R19 H oder CₙH₂ₙ₊₁;
n 1, 2, 3 oder 4;
wobei in CₙH₂ₙ₊₁ ein oder mehrere H-Atome durch F-Atome ersetzt sein können; oder
R17 und R18 gemeinsam mit dem N-Atom, an das sie gebunden sind, einen 5-, 6- oder 7-gliedrigen Ring;
oder
R19 und eine CH₂-Gruppe von R17 oder R18 gemeinsam mit dem N-Atom, an das sie gebunden sind, einen 5- oder 6-gliedrigen Ring;
- R5 und R6: unabhängig voneinander CₚH₂ₚ₊₁, CₛₛH₂ₛₛ₋₁, COR20 oder SO₂R20;
p 1, 2, 3, 4, 5, 6, 7 oder 8,
ss 3, 4, 5, 6, 7 oder 8,
R20 C_{q}H_{2q+1;}
q 1, 2, 3, 4, 5, 6, 7 oder 8,
wobei in den Gruppen CₚH₂ₚ₊₁, CₛₛH₂ₛₛ₋₁ und C_{q}H_{2q+1} ein oder mehrere H-Atome durch F-Atome ersetzt sein können, und eine oder mehrere CH₂-Gruppen durch O oder NR21 ersetzt sein können;
R21 H oder CᵣH₂ᵣ₊₁;
r 1, 2, 3 oder 4;
wobei in CᵣH₂ᵣ₊₁ ein oder mehrere H-Atome durch F-Atome ersetzt sein können;
- R7: H, F, Cl, Br, I, C_{S}H₂ₛ₊₁, C_{dd}H_{2dd-1}, OH, OCₜH₂ₜ₊₁ oder OCOR22;
s und t unabhängig voneinander 1, 2, 3, 4, 5, 6, 7 oder 8;
dd 3, 4, 5, 6, 7 oder 8, wobei in CₛH₂ₛ₊₁, C_{dd}H_{2dd-1} und OCₜH₂ₜ₊₁ ein oder mehrere H-Atome durch F-Atome ersetzt sein können;
R22 CᵤH₂ᵤ₊₁
u 1, 2, 3 oder 4;
wobei in CᵤH₂ᵤ₊₁ ein oder mehrere H-Atome durch F-Atome ersetzt sein können;
- R8, R9 und R10: unabhängig voneinander -Oᵥ-SO_{w}-R23;
v Null oder 1;
w Null, 1 oder 2;
R23 CₙₙH₂ₙₙ₊₁, CₘₘH₂ₘₘ₋₁, OH, OCₚₚH₂ₚₚ₊₁ oder NR25R26;
nn und pp unabhängig voneinander 1, 2, 3, 4, 5, 6, 7 oder 8,
mm 3, 4, 5, 6, 7 oder 8,
wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN oder C_{Z}H_{2z+1}, C_{zz}H_{2zz-1} ;
z 1, 2, 3, 4, 5, 6, 7 oder 8;
zz 3, 4, 5, 6, 7 oder 8, wobei ein oder mehrere H-Atome durch F-Atome ersetzt sein dürfen und in C_{z}H_{2z+1} ein oder mehrere H-Atome durch F-Atome ersetzt sein können und ein oder mehrere CH₂₋Gruppen durch O, CO, CS oder NR27 ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5-, 6- oder 7-gliedrigen Ring,
oder
R27 und eine CH₂-Gruppe von R25 oder R26 gemeinsam mit dem N-Atom, an das sie gebunden sind, einen 5- oder 6-gliedrigen Ring; oder
- R8, R9 und R10: unabhängig voneinander NR32COR30, NR32CSR30 oder NR32SO_{bb}R30;
R30 H, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 unabhängig voneinander H oder CₕH₂ₕ₊₁;
bb 2 oder 3;
cc 1, 2, 3, 4, 5, 6, 7 oder 8;
yy 3, 4, 5, 6, 7 oder 8;
h 1, 2, 3, 4, 5, 6, 7 oder 8, wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂₋Gruppen durch O oder NR31 ersetzt sein können;
R31 H, CₖₖH₂ₖₖ₊₁, COR65;
kk 1, 2, 3, oder 4;
wobei ein oder mehrere H-Atome durch F-Atome ersetzt sein können,
R65 H, CₓₓH₂ₓₓ₊₁; oder
xx 1, 2, 3 oder 4, wobei ein oder mehrere H-Atome durch F-Atome ersetzt sein dürfen; oder
R31 zusammen mit einer CH₂-Gruppe von R30 einen 5-, 6- oder 7-gliedrigen Ring bildet;
R30 ein 5- oder 6-gliedriges Heteroaryl mit 1, 2, 3 oder 4 N-Atomen, Null oder 1 S-Atomen und Null oder 1 O-Atomen,
das unsubstituiert ist oder substituiert mit bis zu drei Substituenten ausgewählt aus der Gruppe bestehend aus F, Cl, Br, l, CₒₒH₂ₒₒ₊₁, NR70R71;
R70 und R71 unabhängig voneinander H, CᵤᵤH₂ᵤᵤ₊₁ und COR72;
R72 H, CᵥᵥH₂ᵥᵥ₊₁;
oo, uu und w unabhängig voneinander 1, 2, 3, 4, 5, 6, 7 oder 8; wobei in den Gruppen CₒₒH₂ₒₒ₊₁, CᵤᵤH₂ᵤᵤ₊₁ oder CᵥᵥH₂ᵥᵥ₊₁ ein oder mehrere H-Atome durch F-Atome ersetzt sein dürfen;
oder
- R8, R9 und R10: unabhängig voneinander H, F, Cl, Br, I, NO₂, CN, OH, NH₂, CₑₑH₂ₑₑ₊₁, C_{ww}H_{2ww-1}. OC_{ff}H_{2ff+1,} NR40R41, CONR40R41, CO0R42, COR42 oder OCOR42,
ee und ff unabhängig voneinander 1, 2, 3, 4, 5, 6, 7 oder 8;
ww 3, 4, 5, 6, 7 oder 8; wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₜₜ₊₁;
tt 1, 2, 3, 4, 5, 6, 7 oder 8; wobei in der Gruppe CₜₜH₂ₜₜ₊₁ ein oder mehrere H-Atome durch F-Atome ersetzt sein dürfen und eine oder mehrere CH₂-Gruppen durch O- oder NR44 ersetzt sein können;
R44 H oder CggH_{2gg+1} ;
gg 1, 2, 3, 4, 5, 6, 7 oder 8; wobei in der Gruppe C_{gg}H_{2gg+1} ein oder mehrere H-Atome durch F-Atome ersetzt sein können, oder
R40 und R41 mit dem N-Atom, an das sie gebunden sind, einen 5- oder 6-gliedrigen Ring;
R42 H oder CₕₕH₂ₕₕ₊₁;
hh 9, 2, 3, 4, 5, 6, 7 oder 8; wobei in der Gruppe CₕₕH₂ₕₕ₊₁ ein oder mehrere H-Atome durch F-Atome ersetzt sein können;
- Y: Fluor, Chlor, Brom, lod, Hydroxy, sowie alle anionischen Formen pharmakologisch verträglicher Mono-, Di- oder Tricarbonsäuren oder Sulfonsäuren;
sowie deren pharmazeutisch berträgliche Salze und Trifluoroacetate;
wobei R2 nicht gleich H ist.

Bevorzugt sind Verbindungen der Formel 1 mit obijer Ausnahme, worin bedeuten:
- R1, R2, R3 und R4: unabhängig voneinander H, F, Cl, Br, I, CN, NO₂, OH, NH₂, CₐH₂ₐ₊₁, Cycloalkyl mit 3, 4, 5 oder 6 C-Atomen, OC_{b}H_{2b+1}, COOR50;
a und b unabhängig voneinander 1, 2, 3 oder 4, wobei ein oder mehrere H-Atome durch F-Atome ersetzt sein können;
R50 H oder C_{c}H_{2c+1};
c 1, 2, 3 oder 4, wobei ein oder mehrere H-Atome durch F-Atome ersetzt sein dürfen;
oder
- R1, R2, R3 und R4: unabhängig voneinander 5- oder 6-gliedriges Heteroaryl, ausgewählt aus der Gruppe bestehend aus Imidazolyl, Pyrazolyl, Pyrrolyl, Triazolyl, Tetrazolyl, Thiazolyl und Oxazolyl;
oder
- R1, R2, R3 und R4: unabhängig voneinander CONR11 R12 oder NR11 R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6, wobei in den Gruppen CₑH₂ₑ₊₁ und CᵣᵣH₂ᵣᵣ₋₁ ein oder mehrere H-Atome durch F-Atome ersetzt sein dürfen oder
R11 und R12 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R11 und R12 gemeinsam mit dem N-Atom, an das sie gebunden sind, einen Pyrrolidin-, Piperidin-, N-Methylpiperazin-, Piperazin- oder Morpholinring;
oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein;
oder
- R1, R2, R3 und R4: unabhängig voneinander OSO₃H, SO₃H, SO₂R₁₅, wobei
R15 CₖH₂ₖ₊₁, OCₗH₂ₗ₊₁ oder NR17R18;
k 1, 2, 3 oder 4, wobei ein oder mehrere H-Atome durch F-Atome ersetzt sein können;
l 1, 2, 3 oder 4, wobei ein oder mehrere H-Atome durch F-Atome ersetzt sein können;
R17 und R18 unabhängig voneinander H, CₘH₂ₘ₊₁ oder CₘH₂ₘ₊₁,in welchem die an den Stickstoff gebundene erste CH₂-Gruppe durch CO und die zweite CH₂-Gruppe durch NR19 ersetzt ist;
m 1, 2, 3, 4 oder 5, wobei in der Gruppe CₘH₂ₘ₊₁ ein oder mehrere H-Atome durch F-Atome ersetzt sein können;
R19 H oder CₙH₂ₙ₊₁;
n 1, 2, 3 oder 4; wobei in CₙH₂ₙ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
- R5 und R6: unabhängig voneinander CₚH₂ₚ₊₁;
p 1, 2, 3 oder 4;
wobei in CₚH₂ₚ₊₁ ein oder mehrere H-Atome durch F-Atome ersetzt sein können;
- R7: H, CₛH₂ₛ₊₁, OCₜH₂ₜ₊₁ oder OCOR22;
s und t unabhängig voneinander 1, 2, 3 oder 4;
wobei in CₛH₂ₛ₊₁ und OCₜH₂ₜ₊₁ ein oder mehrere H-Atome durch F-Atome ersetzt sein können;
R22 CᵤH₂ᵤ₊₁;
u 1, 2, 3 oder 4; wobei in CᵤH₂ᵤ₊₁ ein oder mehrere H-Atome durch F-Atome ersetzt sein können;
- R8, R9 und R10: unabhängig voneinander OSO₃H, SO₃H oder SO₂R23;
R23 CₙₙH₂ₙₙ₊₁, CₘₘH₂ₘₘ₋₁, OCₚₚH₂ₚₚ₊₁ oder NR25R26;
nn und pp unabhängig voneinander 1, 2, 3, 4 oder 5,
mm 3, 4, 5 oder 6, wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN, C_{z}H_{2z+1} oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein;
R27 H oder CₐₐH₂ₐₐ₊₁ ;
aa 1, 2, 3 oder 4; wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring; oder
R8, R9 und R10 unabhängig voneinander NR32COR30, NR32CSR30 oder NR32SO₂R30;
R30 H, OH, C_{cc}H_{2cc+1}, C_{yy}H_{2yy-1}; Pyrrolidinyl oder Piperidinyl, in welchen Ringen eine CH₂-Gruppe durch O oder NR33 ersetzt sein kann;
R32 und R33 unabhängig von einander H oder CₕH₂ₕ₊₁;
cc 1, 2, 3, 4, 5 oder 6;
yy 3, 4, 5 oder 6;
h 1, 2, 3 oder 4; wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂₋Gruppen durch O oder NR31 ersetzt sein können;
R31 H, CₖₖH₂ₖₖ₊₁ oder COR65;
kk 1, 2, 3, oder 4; wobei ein oder mehrere H-Atome durch F-Atome ersetzt sein können,
R65 H, CₓₓH_{2xx+1;}
xx 1, 2, 3 oder 4, wobei ein oder mehrere H-Atome durch F-Atome ersetzt sein dürfen; oder
R31 zusammen mit einer CH₂-Gruppe von R30 und dem N-Atom, an das sie gemeinsam gebunden sind einen 5- oder 6-gliedrigen Ring bilden; oder
R30 einen 5- oder 6-gliedrigen Heteroaromaten ausgewählt aus der Gruppe bestehend aus Pyridyl, Imidazolyl, Pyrazolyl, Pyrrolyl, Triazolyl, Tetrazolyl, Thienyl, Thiazolyl und Oxazolyl,
die unsubstituiert ist oder substituiert mit bis zu drei Substituenten ausgewählt aus der Gruppe bestehend aus F, Cl, Br, I, CₒₒH₂ₒₒ₊₁, NR70R71,
R70 und R71 unabhängig voneinander H, CᵤᵤH₂ᵤᵤ₊₁ oder COR72,;
R72 H, CᵥᵥH₂ᵥᵥ₊₁;
oo, uu und vv unabhängig voneinander 1, 2, 3 oder 4; wobei in den Gruppen CₒₒH₂ₒₒ₊₁, CᵤᵤH₂ᵤᵤ+1 oder CᵥᵥH₂ᵥᵥ₊₁ ein oder mehrere H-Atome durch F-Atome ersetzt sein dürfen;
oder
- R8, R9 und R10: unabhängig voneinander H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42;
ee und ff unabhängig voneinander 1, 2, 3 oder 4;
ww 3, 4, 5 oder 6, wobei in den Gruppen CₑₑH₂ₑₑ₊₁, C_{ww}H_{2ww-1} und OC_{ff}H₂f_{f+1} ein oder mehrere H-Atome durch F-Atome ersetzt sein können;
R40 und R41 H oder CₜₜH₂ₜₜ₊₁;
tt 1, 2, 3, 4, 5, 6, 7 oder 8; wobei in der Gruppe CₜₜH₂ₜₜ₊₁ ein oder mehrere H-Atome durch F-Atome ersetzt sein dürfen;
oder
R40 und R41 unabhängig voneinander auszuwählen Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können;
- Y: Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

Besonders bevorzugt sind Verbindungen der Formel I mit obiger Ausnahme, worin bedeuten:
- R1, R2, R3 und R4: unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
- R1, R2, R3 und R4: unabhängig voneinander NR11 R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen; oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin; oder
R11 und R12 unabhängig voneinander COR14, CSR14, CONHR14, CSNHR14 oder S0₂R14;
R14 C_{g}H_{2g+1};
g 1, 2, 3 oder 4, wobei ein oder mehrere H-Atome durch F-Atome ersetzt sein können;
oder
- R1, R2, R3 und R4: unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
- R5 und R6: unabhängig voneinander Methyl oder Trifluormethyl;
- R7: H;
- R8, R9 und R10: unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2z+1}, oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R8, R9 und R10: unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5 oder 6;
yy 3, 4, 5 oder 6; wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch O oder NR31 ersetzt sein können;
R31 H, Methyl, Ethyl, CF₃, CH₂CF₃, Acetyl oder Propionyl; oder
R31 zusammen mit einer CH₂-Gruppe von R30 und dem N-Atom, an das sie gemeinsam gebunden sind, einen 5- oder 6-gliedrigen Ring bilden; oder
R30 Pyridyl, Imidazolyl, Pyrazolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
- R8, R9 und R10: unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₜₜ₊₁;
tt 1, 2, 3 oder 4; wobei in der Gruppe CₜₜH₂ₜₜ₊₁ ein oder mehrere H-Atome durch F-Atome ersetzt sein dürfen;
oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁ ;
hh 1, 2, 3 oder 4; wobei in der Gruppe CₕₕH₂ₕₕ₊₁ ein oder mehrere H-Atome durch F-Atome ersetzt sein können;
- Y: Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

Ganz besonders bevorzugt sind Verbindungen der Formel I mit obiger Ausnahme, worin bedeuten:
- R1 und R3: H;
- R2 und R4: unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ+₁ und OC_{b}H_{2b+1} unabhängig voneinander 1, 2, 3 oder 4, wobei ein oder mehrere H-Atome durch F-Atome ersetzt sein können;
oder
- R2 und R4: unabhängig voneinander NR11R12;
R11 und R12 unabhängig voneinander H, CₑH₂ₑ₊₁,
e 1, 2, 3 oder 4,
wobei in CₑH₂ₑ₊₁ ein oder mehrere H-Atome durch F-Atome ersetzt sein dürfen; oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin; oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
- R2 und R4: unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3,4 oder 5, wobei in der Gruppe CₘH₂ₘ₊₁ ein oder mehrere H-Atome durch F-Atome ersetzt sein können; oder
R17 und R18 gemeinsam mit dem N-Atom, an das sie gebunden sind, einen 5- oder, 6-gliedrigen Ring;
- R5 und R6: unabhängig voneinander Methyl oder Trifluormethyl;
- R7: H;
- R8, R9 und R10: unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2Z+1}, oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁;
aa 1, 2, 3 oder 4;
wobei in CₐₐH₂ₐₐ₊₁ ein oder mehrere H-Atome durch F-Atome ersetzt sein können; oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
- R8, R9 und R10: unabhängig voneinander NR32COR30, NR32CSR30 oder NR32SO₂R30;
R30 H, OH, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5 oder 6;
yy 3, 4, 5 oder 6; wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch O oder NR31 ersetzt sein können;
R31 H, Methyl, Ethyl, CF₃, CH₂CF₃, Acetyl oder Propionyl; oder
R31 zusammen mit einer CH₂-Gruppe von R30 und dem N-Atom, an das sie gemeinsam gebunden sind, einen 5- oder 6-gliedrigen Ring bilden; oder
R30 Pyridyl, Imidazolyl, Pyrazolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
- R8, R9 und R10: unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₜₜ₊₁;
tt 1, 2, 3 oder 4; wobei in der Gruppe CₜₜH₂ₜₜ₊₁ ein oder mehrere H-Atome durch F-Atome ersetzt sein dürfen; oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl; oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁;
hh 1, 2, 3 oder 4; wobei in der Gruppe CₕₕH₂ₕₕ₊₁ ein oder mehrere H-Atome durch F-Atome ersetzt sein können;
- Y: Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

Außerordentlich ganz besonders bevorzugt sind die folgenden Tetrahydroisochinolinium-Salze:
a. 6,8-Dichloro-2,2-dimethyl-4-phenyl-1,2,3,4-tetrahydro-isochinolinium-trifluoracetat;
b. 6,8-Dichloro-2,2-dimethyl-4-(4-sulfamoyl-phenyl)-1,2,3,4-tetrahydro-isochinolinium-trifluoracetat;
c. 4-(4-Acetylamino-phenyl)-6,8-dichloro-2,2-dimethyl-1,2,3,4-tetrahydroisochinolinium-iodid;
d. (+)-4-(4-Acetylamino-phenyl)-6,8-dichloro-2,2-dimethyl-1,2,3,4-tetrahydroisochinolinium-iodid;
e. (-)-4-(4-Acetylamino-phenyl)-6,8-dichloro-2,2-dimethyl-1,2,3,4-tetrahydroisochinolinium-iodid;
f. (+)-4-(4-Acetylamino-phenyl)-6,8-dichloro-2,2-dimethyl-1,2,3,4-tetrahydroisochinolinium-chlorid;
g. 4-(4-Amino-phenyl)-6,8-dichloro-2,2-dimethyl-1,2,3,4-tetrahydro-isochinolinium-chlorid; Hydrochlorid;
h. 6,8-Dichloro-4-[4-(3-ethyl-ureido)-phenyl]-2,2-dimethyl-1,2,3,4-tetrahydroisochinolinium-chlorid;
sowie deren pharmazeutisch verträgliche Salze.

Die bezeichneten Alkylreste, bzw. teilweise oder vollständig fluorierten Alkylreste können sowohl geradkettig als auch verzweigt vorliegen. Gruppen CₐH₂ₐ₋₁ bzw. deren Analoga bis C_{yy}H_{2yy-1} bedeuten entweder die entsprechenden Alkenyle, Cycloalkyle, Cycloalkyl-alkyle oder Alkyl-cycloalkyle.

Als Heteroaryle gelten insbesondere 2- oder 3-Thienyl, 2- oder 3-Furyl, 1-, 2- oder 3-Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-indazolyl, 2-, 3-, 4-, 5-, 6-, 7- oder 8-Chinolyl, 1-, 3-, 4-, 5-, 6-, 7- oder 8-lsochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also z.B. 1-Oxy-2-, 3- oder 4-pyridyl.

Bevorzugt sind dabei die 5- oder 6-gliedrigen Heterocyclen davon.

Insbesondere bevorzugt sind die Heterocyclen Imidazolyl, Pyrazolyl, Pyridyl, Pyrrolyl, Triazolyl, Tetrazolyl, Thiazolyl und Oxazolyl

Als pharmakologisch verträgliche Anionen Y kommen diejenigen von folgenden Mono-, Di- oder Tricarbonsäuren oder Sulfonsäuren in Frage: Essigsäure, Adipinsäure, Zitronensäure, Bernsteinsäure, Äpfelsäure, Fumarsäure, Gluconsäure, Glutaminsäure, Glycerolphosphorsäure, HCl, HBr, Milchsäure, Malonsäure, Maleinsäure, Methansulfonsäure, Ethansulfonsäure , Salpetersäure, Di-(2-hydroxy-3-carbonsäure-naphth-1-yl)-methan (Pamoate), Phosphorsäure, Schwefelsäure, Weinsäure, Toluolsulfonsäure

Bei mehrfach negativ geladenen Säure-Anionen Y können ein oder mehrere Kationen nach der Erfindung vorhanden sein.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Als CH₂-Einheiten gelten auch die in einer Alkylkette terminalen CH₃-Gruppen, die in diesem Zusammenhang als CH₂-H Gruppierungen aufgefasst werden.

Als pharmakologisch bzw. physiologisch oder toxikologisch verträgliche Salze der Verbindungen der Formel 1 kommen in Frage: die Alkalimetallsalze, vorzugsweise Natrium- oder Kaliumsalze, oder die Erdalkalimetallsalze, z. B. Calcium- oder Magnesiumsalze, oder die Ammoniumsalze, z. B. als Salze mit Ammoniak oder organischen Aminen oder Aminosäuren. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw.

Besonders die Salze der folgenden Säuren werden sehr sind sehr gut geeignet: Maleinsäure, Fumarsäure, Bernsteinsäure, Äpfelsäure, Weinsäure, Methylsulfonsäure, Salzsäure, Schwefelsäure, Phosphorsäure

In den Offenlegungsschriften WO 01 32 624 und WO 01 32 625 sind Verbindungen des Typs I, jedoch nicht als quarternäre Verbindungen, als Reuptake-Inhibitoren von Norepinephrin, Dopamin und Serotonin beschrieben. Allerdings sind in diesen Patentanmeldungen ausschließlich Verbindungen geschützt, bei denen R1 und R2 ausschließlich H sein dürfen. Bei den erfindungsgemäßen Verbindungen hat sich allerdings gezeigt, dass zumindest für R2 gelten muss, dass R2 nicht gleich H ist. Des weiteren konnte anhand einer Beispielverbindung der erfindungsgemäßen Verbindungen keine inhibitorischen Eigenschaften an den beschriebenen Rezeptoren nachgewiesen werden, so dass sich die beschriebenen Verbindungen sowohl strukturell als auch in ihren pharmakologischen Eigenschaften von den in den erwähnten Patentanmeldungen beschriebenen Verbindungen deutlich unterscheiden.

Des weiteren sind Verbindungen des Typs I, ebenfalls nicht als quarternäre Verbindungen, in der Offenlegungsschrift EP 11 13 007 als Östrogenagonisten und -antagonisten beschrieben. Es konnte gezeigt werden, dass die erfindungsgemäßen Verbindungen keine Aktivität an den genannten Rezeptoren zeigen, so dass auch hier die strukturellen Unterschiede der erfindungsgemäßen Verbindungen in deutlich anderen pharmakologischen Eigenschaften resultieren.

In der Offenlegungsschrift WO03048129 (DEAV2001/0072) wird die Verwendung der 4-Phenyltetrahydroisochinoline als NHE-Inhibitoren vorgeschlagen. Es hat sich nun gezeigt, dass die analogen quartären Ammoniumverbindungen ebenso exzellente NHE-Inhibitoren sind, insbesondere inhibieren sie den NHE3. Die erfindungsgemäßen Verbindungen zeichnen sich aber darüber hinaus durch eine deutlich höhere Wasserlöslichkeit aus, was zu einer verbesserten Nierengängigkeit führen sollte.

Die Publikationen J. Med. Chem. 1984, 27, 28-35, J. Med. Chem. 1990, 33, 2286-2296 und Dtsch. Apoth.-Ztg. 1977,117,17, 611-614 offenbaren die Einzelverbindungen 8-Amino-4-(3,4-dihydroxy-phenyl)-2,2-dimethyl-1,2,3,4-tetrahydro-isoquinolinium iodid, 4-Hydroxy-2,2-dimethyl-4-phenyl-1,2,3,4-tetrahydro-isoquinolinium iodid und 8-Amino-2,2-dimethyl-4-phenyl-1,2,3,4-tetrahydro-isoquinolinium iodid, betreffen jedoch andere technische Gebiete. DE10019062 beschreibt 2-Guanidino-4-aryl-chinazoline und EP659748 substituierte 1-Oxo-1,2-dihydro-isochinolinoyl- und 1,1-Dioxo-2H-1,2-benzothiazinoylguanidine.

Beschrieben werden auch Methoden zur Herstellung der verwendeten Verbindungen. Die Synthese der analogen Tetrahydroisochinoline II ist in der deutschen Patentanmeldung 101 59 714.2 (DEAV2001/0072) bereits beschrieben:

So lassen sich die erfindungsgemäßen Verbindungen ausgehend von den Benzylaminvorläufern IV herstellen. Diese wiederum können, falls nicht käuflich erhältlich, nach Standardverfahren aus den entsprechenden Benzylchloriden oder Benzylbromiden lll synthetisiert werden.

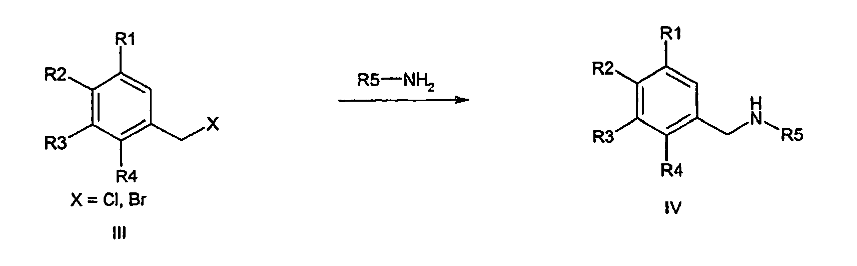

Die so erhaltenen Benzylamine IV, werden in dem Fachmann bekannter Weise mit den entsprechend substituierten alpha-Bromacetophenonverbindungen V alkyliert.

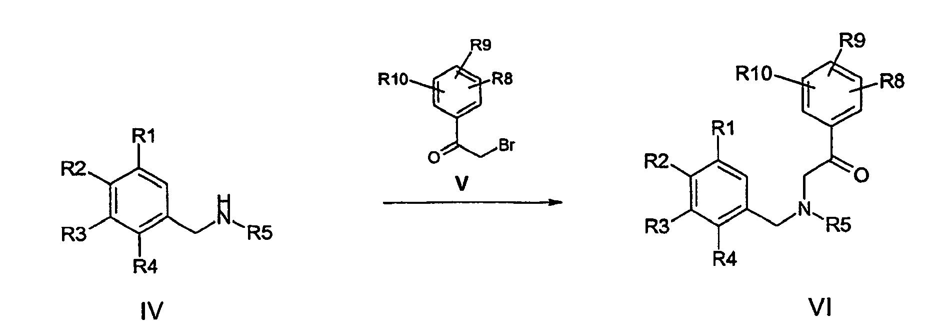

Die alpha-Bromacetophenonverbindungen V lassen sich in literaturbekannten Verfahren aus den entsprechenden Acetophenonvorläufern durch Bromierung gewinnen. Durch Reduktion der Carbonylgruppierung in VI und anschließender säurekatalysierter Cyclisierung der entsprechenden Alkohole VII können die gewünschten Tetrahydroisochinoline 11 nach bekannten Verfahren gewonnen werden (vgl. Tetrahedron Lett.; 1989, 30, 5837; Org. Prep. Proced. lnt.; 1995, 27, 513; J. Med. Chem.; 1973, 16, 342).

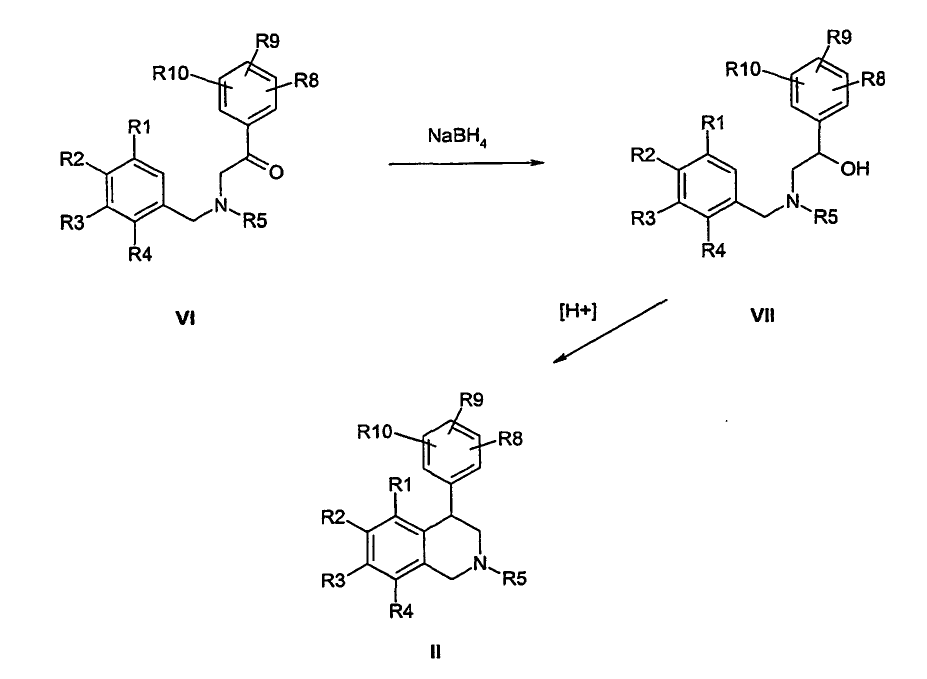

Für R7 ungleich H lassen sich die gewünschten Verbindungen der Formel II z. B. aus den lodiden VIII durch Halogen-Metall-Austausch und anschließendem nucleophilen Angriff der intermediären lithiumorganischen Spezies an der Carbonylgruppe herstellen (vgl. Chem. Pharm. Bull.; 1995, 43, 1543).

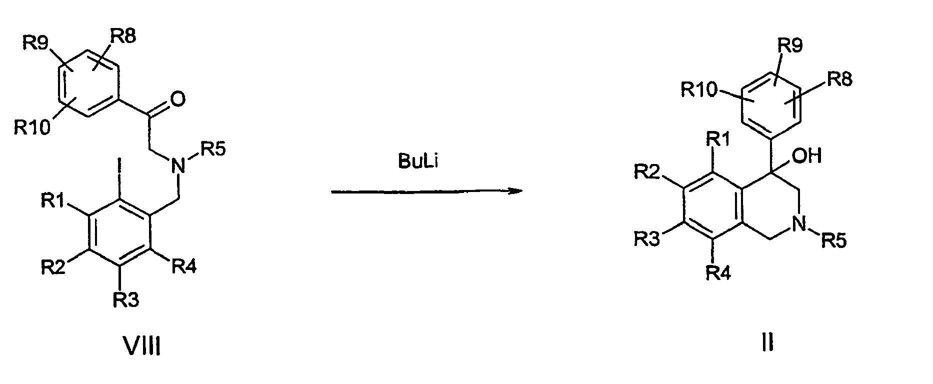

Die dabei synthetisierten tertiären Alkohole 11 lassen sich durch bekannte Methoden in weitere Derivate überführen.
Zur Herstellung alkylverzweigter Analoge (II) werden die entsprechenden Diphenylessigsäureester X in alpha-Stellung nach bekannten Methoden alkyliert. Die gewünschten Produkt XI können durch Standardverfahren in die entsprechenden Amide XII überführt werden, welche in einer Pictet-Spengler-analogen Reaktion in die gewünschten Tetrahydroisochinoline II überführt werden (vgl. Tetrahedron; 1987, 43, 439; Chem. Pharm. Bull.; 1985, 33, 340).

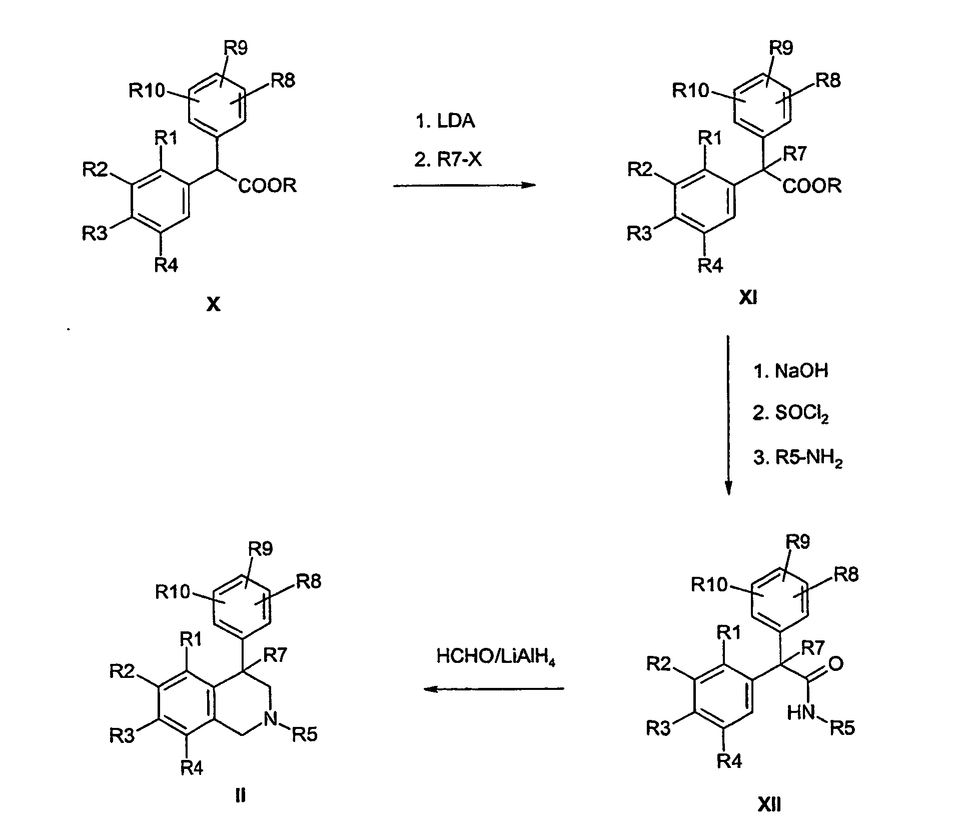

Ausgehend von II können die erfindungsgemäßen Verbindungen in dem Fachmann bekannter Weise durch Alkylierungsreaktionen hergestellt werden.

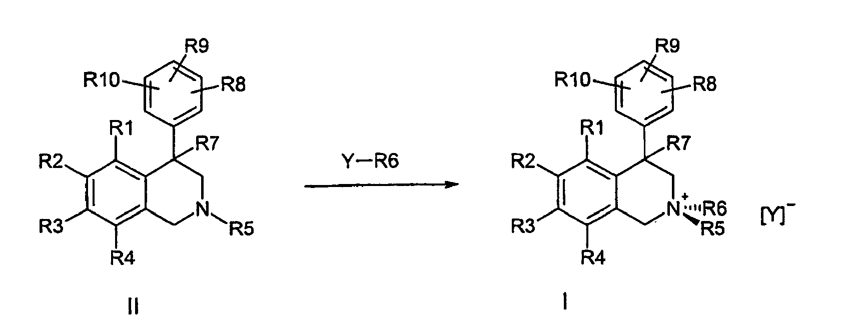

Als Alkylierungsreagenzien lassen sich die entsprechenden Halogenide, Methansulfonate, Trifluormethansulfonate oder auch Tosylate verwenden. Die bei der Reaktion entstandenen Tetrahydroisochinolinium-Salze lassen sich durch lonenaustauschchromatographie in bekannter Weise leicht in andere Salze überführen.

Es konnte gezeigt werden, dass Verbindungen der Formel I hervorragende Inhibitoren des Natrium-Wasserstoffaustauschers (NHE) - insbesondere des Natrium-Wasserstoffaustauschers vom Subtyp 3 (NHE3) - darstellen.

Aufgrund dieser Eigenschaften eignen sich die Verbindungen zur Behandlung von Krankheiten, die durch Sauerstoffmangel hervorgerufen werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindem. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺⁻Austauschmechanismus als Arzneimittel zur Behandlung aller akuten oder chronischen durch lschämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Himödems geeignet sind. Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.

Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäß verwendeten Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel 1 wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikaments zur Behandlung diabetischer Spätschäden und chronischer Nierenerkrankungen, insbesondere von allen Nierenentzündungen (Nephritiden), die mit einer vermehrten Protein-/ Albuminausscheidung verbunden sind; zur Herstellung eines Medikaments zur Behandlung des Befalls durch Ektoparasiten in der Human- und Veterinärmedizin; zur Herstellung eines Medikaments zur Behandlung der genannten Leiden in Kombinationen mit blutdrucksenkenden Stoffen, bevorzugt mit Angiotensin Converting Enzyme (ACE)-Hemmem, mit Diuretika und Saluretika wie Furosemid, Hydrochlorothiazid, Pseudoaldosteronantagonisten und Aldosteron-Antagonisten; mit Adenosinrezeptor-Modulatoren, insbesondere mit Adenosinrezeptor-Aktivatoren (A2-Agonisten); und mit Angiotensin-Rezeptorantagonisten.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmem mit blutdrucksenkenden und/oder hypolipidämisch wirkenden Arzneimitteln.

Arzneimittel, die eine Verbindung I enthalten, können dabei oral, parenteral, intravenös, rektal, transdermal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgem können beispielsweise Antioxidantien, Dispergiermittel, Emulgatoren, Entschäumer, Geschmackskorrigentien, Konservierungsmittel, Lösungsvermittler oder Farbstoffe verwendet werden.
Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Versuchsbeschreibungen und Beispiele:

Liste der verwendeten Abkürzungen:
- Rₜ: Retentionszeit
- TFA: Trifluoressigsäure
- HPLC: High Performance Liquid Chromatography
- eq: Äquivalente
- LCMS: Liquid Chromatography Mass Spectroscopy
- MS: Mass Spectroscopy
- Cl: Chemical lonization
- RT: Raumtemperatur
- THF: Tetrahydrofuran
- TOTU: O-[(Ethoxycarbonyl)-cyanomethylenamino]-N,N,N',N'-tetramethyluronium-tetrafluoroborat
- DMSO: Dimethylsulfoxid
- abs.: absolut(es)
- Zers.: Zersetzung

### Allgemeines:

Die im Folgenden angegebenen Retentionszeiten (Rₜ) beziehen sich auf LCMS-Messungen mit den folgenden Parametern:

### Methode A:

- stationäre Phase:: Merck Purospher 3µ2 x 55 mm
- mobile Phase:: 95% H₂O (0.05% TFA)→ 95% Acetonitril; 4 min; 95% Acetonitril; 1.5 min → 5% Acetonitril; 1 min; 0.5 ml/min.

### Methode A1:

- stationäre Phase:: Merck Purospher 3µ2 x 55 mm
- mobile Phase:: 95% H₂O (0.05% TFA)→ 95% Acetonitril; 3 min; 95% Acetonitril; 1.5 min → 5% Acetonitril; 1 min; 0.5 ml/min.

### Methode B:

- stationäre Phase:: Merck Purospher 3µ2 x 55 mm
- mobile Phase:: 0 min 90% H₂O (0.05% TFA) 2,5 min-95 % Acetonitril; 95 % Acetonitril bis 3,3 min; 10 % Acetonitril 3,4 min; 1 ml/min.

### Methode C:

- stationäre Phase:: Merck Purospher 50 x 2,1 mm
- mobile Phase:: 95% H₂O (0.1 % HCOOH)→ 95% Acetonitril; 5 min; 95% Acetonitril; 7 min; 0.45 ml/min.

Die Retentionszeiten beziehen sich auf die MS-Spektren.

### Beispiel 1:

6,8-Dichloro-2,2-dimethyl-4-phenyl-1,2,3,4-tetrahydro-isochinolinium, trifluoracetat;

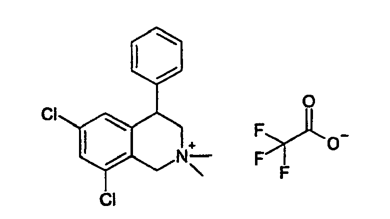

### Zwischenprodukt 1:

2,4-Dichlorbenzylmethylamin wird nach literaturbekannten Methoden hergestellt (J. Med. Chem.; 1984, 27, 1111).

### Zwischenprodukt 2:

2-[(2,4-Dichloro-benzyl)-methyl-amino]-1-phenyl-ethanon;
14,1.g (74,2 mmol) des Zwischenprodukts 1 werden in 100 ml Dioxan gelöst und bei Raumtemperatur tropfenweise mit einer Lösung bestehend aus 16,9 g (89 mmol) 2-Bromacetophenon in 100 ml Dioxan versetzt. Anschließend werden 51,2 ml (370 mmol) Triethylamin zugegeben und vier Stunden bei Raumtemperatur gerührt. Nach Stehen über Nacht wird vom entstandenen Niederschlag abgesaugt und vom Lösungsmittel befreit. Der Rückstand wird in Essigester gelöst und mit 2 N HCl, H₂O und NaHCO₃-Lösung gewaschen. Die HCl-Phase wird mit KOH auf einen pH von 12 eingestellt und zweimal mit Essigester extrahiert. Die vereinigten organischen Phasen werden mit Na₂SO₄ getrocknet und eingeengt. Chromatographie an Kieselgel liefert 20,6 g der Titelverbindung als gelbes Öl (Rt = 4,188 min(Methode A); MS(CI⁺) = 308,2/310,2).

### Zwischenprodukt 3:

2-[(2,4-Dichloro-benzyl)-methyl-amino]-1-phenyl-ethanol;
Zwischenprodukt 2 (20,6 g; 66,9 mmol) wird in 150 ml abs. Methanol gelöst und bei 0 °C portionsweise mit 5,06 g (133,8 mmol) Natriumborhydrid versetzt. Es wird zwei Stunden bei Raumtemperatur gerührt. Zur Aufarbeitung wird das Lösungsmittel i. Vak. entfernt, der Rückstand in Essigester aufgenommen und zweimal mit H₂O gewaschen.
Die Essigesterphase wird mit Na₂SO₄ getrocknet und eingeengt, wobei 20 g Rohprodukt erhalten werden, die ohne weitere Reinigung weiter umgesetzt werden können (Rt = 4,149 min(Methode A); MS(Cl⁺) = 310,2/312,2).

### Zwischenprodukt 4:

6,8-Dichloro-2-methyl-4-phenyl-1,2,3,4-tetrahydro-isochinolin;
20 g (64,5 mmol) des Zwischenprodukts 3 werden in 55 ml Dichlormethan gelöst und auf 0°C gekühlt. Diese Lösung wird zu 55 ml einer vorgekühlten konz. H₂SO₄ getropft und im Anschluss zwei Stunden bei Raumtemperatur gerührt. Zur Aufarbeitung gibt man auf Eis und stellt mit 6 N NaOH stark alkalisch. Es wird dreimal mit Dichlormethan extrahiert. Die vereinigten organischen Phasen werden mit MgSO₄ getrocknet und eingeengt. Das ölige Rohprodukt wird an Kieselgel gereinigt, wobei Zwischenprodukt 4 in 53 % Ausbeute erhalten wird (Rt = 4,444 min(Methode A); MS(Cl⁺) = 292,2/294,2).

6,8-Dichloro-2,2-dimethyl-4-phenyl-1,2,3,4-tetrahydro-isochinolinium, trifluor-acetat; Zwischenprodukt 4 (100 mg) wurde in Form seines Hydrochlorids in Gegenwart von Diisopropylethylamin (58 µl) in Aceton (1 ml) gelöst. Anschließend wurde unter Rühren Methyljodid (38 µl) zugetropft. Das Gemisch wurde dann zwei Stunden bei Raumtemperatur gerührt und anschließend 62 h stehen gelassen. Da die Umsetzung noch nicht vollständig war, wurde weiteres Methyljodid (38 µl) zugegeben und das Gemisch zum Rückfluss erhitzt. Nach zwei Stunden wurde das Reaktionsgemisch abgekühlt und anschließend am Rotationsverdampfer zur Trockene eingeengt. Der Rückstand wurde mittels präparativer HPLC an RP-18 mit Acetonitril/Wasser (0,05% TFA) gereinigt und die sauberen Fraktionen vereinigt. Nach Abziehen des Acetonitrils wurde der wässrige Rückstand gefriergetrocknet. Es wurden 132 mg des gewünschten Produkts als Feststoff erhalten. (Rₜ = 4,22 min(Methode A); MS (Cl⁺): 306,0).

### Beispiel 2:

6,8-Dichloro-2,2-dimethyl-4-(4-sulfamoyl-phenyl)-1,2,3,4-tetrahydro-isochinolinium, trifluoracetat;

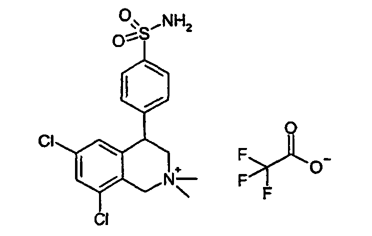

### Zwischenprodukt 1

4-(6,8-Dichloro-2-methyl-1,2,3,4-tetrahydro-isochinolin-4-yl)-benzolsulfonamid; Zu 10 ml (150 mmol) Chlorsulfonsäure wird bei 0°C eine Lösung von 3,0 g (10 mmol) 6,8-Dichloro-2-methyl-4-phenyl-1,2,3,4-tetrahydro-isochinolin (Zwischenprodukts 4, Beispiel 1) in 30 ml Dichlormethan langsam zugetropft. Es wird eine Stunde bei 0 °C und eine Stunde bei Raumtemperatur gerührt. Zur Aufarbeitung wird der Reaktionsansatz auf Eis gegeben und mit gesättigter NaHCO₃-Lösung ein pH-Wert von 8 eingestellt.. Es wird dreimal mit Essigester extrahiert und die organischen Phasen mit Na₂SO₄ getrocknet und eingeengt. Das so erhaltene Rohprodukt (3,34 g) wird in 200 ml konz. Ammoniak suspendiert und auf 90 °C erhitzt. Nach drei Stunden wird das Lösungsmittel abdestilliert und der Rückstand in wenig H₂O aufgenommen und dreimal mit Essigester extrahiert. Die vereinigten Essigesterphasen werden mit Na₂SO₄ getrocknet und eingeengt, wobei 2,76 g eines amorphen Rohprodukts erhalten werden. Zur weitere Reinigung wird an einer Kieselgelsäule getrennt (Dichlormethan / Methanol 95:5), wobei 830 mg des gewünschten Sulfonamids erhalten werden.

### 6,8-Dichloro-2,2-dimethyl-4-(4-sulfamoyl-phenyl)-1,2,3,4-tetrahydro-isochinolinium, Trifluoracetat;

371 mg (1,0 mmol) des Zwischenprodukts 1 werden in 10 ml DMF gelöst und bei Raumtemperatur mit 11 eq. Methyliodid versetzt. Nach drei Stunden wird vom Lösungsmittel befreit und der Rückstand in H₂O digeriert. Nach Trocknen über P₂O₅ werden 380 mg Rohprodukt erhalten, das an einer präparativen HPLC gereinigt wird. (Rt = 3,583 min(Methode A); MS(ES⁺) = 385,0/387,0).

### Beispiel 3:

3a: 4-(4-Acetylamino-phenyl)-6,8-dichloro-2,2-dimethyl-1,2,3,4-tetrahydroisochinolinium-iodid; 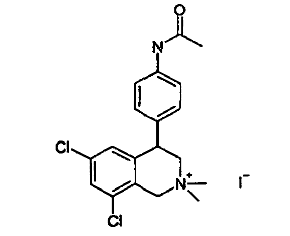
3b: (+)-4-(4-Acetylamino-phenyl)-6,8-dichloro-2,2-dimethyl-1,2,3,4-tetrahydroisochinolinium-iodid; 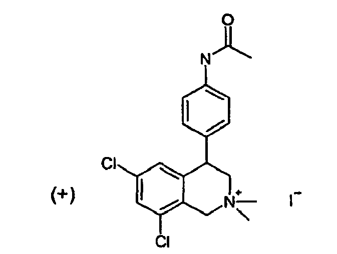
3c: (+)-4-(4-Acetylamino-phenyl)-6,8-dichloro-2,2-dimethyl-1,2,3,4-tetrahydroisochinolinium-chlorid; 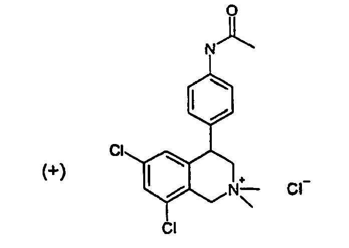
3d: (-)-4-(4-Acetylamino-phenyl)-6,8-dichloro-2,2-dimethyl-1,2,3,4-tetrahydroisochinolinium-iodid;

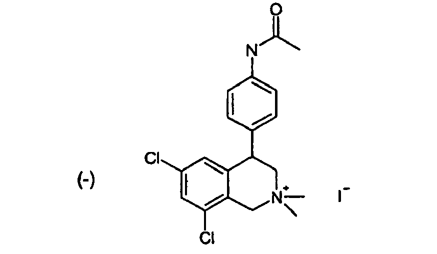

### Zwischenprodukt 1:

2,4-Dichlorbenzylmethylamin wird nach literaturbekannten Methoden hergestellt (J. Med. Chem.; 1984, 27, 1111).

### Zwischenprodukt 2:

N-[4-(2-Bromo-acetyl)-phenyl]-acetamid wird in dem Fachmann bekannter Weise durch Bromierung von N-(4-Acetyl-phenyl)-acetamid synthetisiert.
Die Ausgangsverbindung (0,256 mol) wird in 300 ml Essigsäure vorgelegt und bei 60°C eine Lösung von 39,9 g Brom (1,0 eq) in 60 ml Essigsäure zugetropft. Nach 1,5 h wird auf Raumtemperatur abkühlen lassen und die Reaktionsmischung auf 1 1 Eiswasser gegeben. Der Niederschlag wird abgesaugt, mit Wasser gewaschen und getrocknet, wobei 60 g der Titelverbindung isoliert werden (Schmp.: 192°C).

### Zwischenverbindung 3:

N-(4-{2-[(2,4-Dichloro-benzyl)-methyl-amino]-acetyl}-phenyl)-acetamid;
37,1 g (0,195 mol) des Zwischenprodukts 1 werden in 400 ml Dioxan vorgelegt und mit einer Lösung aus 60 g (0,234 mol) des Zwischenprodukts 2 in 600 ml Dioxan versetzt. Es werden 134 ml Triethylamin zugegeben und 4 h bei Raumtemperatur gerührt. Nach Stehen über Nacht wird der Niederschlag abfiltriert und das Filtrat i. Vak. eingeengt. Der Rückstand wird in Essigester aufgenommen, mit NaHCO₃ und H₂O gewaschen, mit MgSO₄ getrocknet und eingeengt. Der hierbei anfallende ölige Rückstand wird mit einem Essigester/Ether-Gemisch verrieben, wobei 36 g des Zwischenprodukt 3 in Form eines kristallinen Feststoffs anfallen (Schmp.: 115-117°C).

### Zwischenprodukt 4:

N-(4-{2-[(2,4-Dichloro-benzyl)-methyl-amino]-1-hydroxy-ethyl}-phenyl)-acetamid;
36 g (0,099 mol) des Zwischenprodukts 3 werden in 500 ml Methanol gelöst und bei 0°C mit 7,8 g (2 eq) Natriumborhydrid versetzt. Es wird noch 30 min bei 0°C und eine weitere Stunde bei Raumtemperatur nachgerührt. Zur Aufarbeitung wird der Reaktionsansatz eingeengt und der Rückstand zwischen 1 N HCl und Essigester verteilt. Die wässrige Phase wird abgetrennt, auf pH 9 eingestellt und zweimal mit Essigester extrahiert. Die vereinigten organischen Phasen werden mit MgSO₄ getrocknet und eingeengt. Das so erhaltene Rohprodukt kann ohne weitere Reinigung weiter umgesetzt werden.

### Zwischenprodukt 5:

N-[4-(6,8-Dichloro-2-methyl-1,2,3,4-tetrahydro-isochinolin-4-yl)-phenyl]-acetamid;
20 g (0,054 mol) der Zwischenverbindung 4 werden in 250 ml Dichlormethan gelöst und bei 0°C mit 250 ml konz. H₂SO₄ tropfenweise versetzt. Es wird zwei Stunden bei 0°C und eine weitere Stunde bei Raumtemperatur gerührt. Zur Aufarbeitung wird die Reaktionsmischung auf Eiswasser gegeben und der Niederschlag abgesaugt. Der Niederschlag wird in 300 ml 1 N NaOH aufgenommen und dreimal mit Essigester extrahiert. Trocknen der organischen Phasen und Einengen liefert ein Rohprodukt, das mit Diisopropylether verrieben wird, wobei 11,7 g der Beispielverbindung als kristalliner Feststoff isoliert werden (Schmp.: 205-206°C).

4-(4-Acetylamino-phenyl)-6,8-dichloro-2,2-dimethyl-1,2,3,4-tetrahydro-isochinolinium-iodid (Beispiel 3a);
2,0 g (5,7 mmol) des Zwischenprodukts 5 werden in 60 ml abs. DMF vorgelegt und bei Raumtemperatur mit 3,6 ml (60,0 mmol) Methyliodid versetzt. Es wird drei Stunden bei Raumtemperatur gerührt und anschließend i. Vak. eingeengt. Der Rückstand wird einmal mit wenig H₂O verrührt abgesaugt und noch einmal mit siedendem Essigester extrahiert. Absaugen liefert 2,65 g eines leicht gelben Feststoffs als Rohprodukt. Davon wurde 1,0 g in 250 ml H₂O gelöst und mit 100 ml Essigester extrahiert. Die wässrige Phase wird filtriert und eingeengt, wobei 837 mg des gewünschten Ammonium-iodids in Form eines farblosen Feststoffs erhalten werden. (Rt = 3,804 min(Methode A); MS(ES⁺) = 363,1/365,1).

2,0 g des Zwischenprodukts 5 wurden an einer chiralen Phase in die Enantiomere getrennt. Bedingungen:
stationäre Phase: Chiralpak AD 250 x 4,6; 20 µm;
moblie Phase Acetonitril
Flußrate: 1 ml/min
Rt(Enantiomer 1) = 5,856 min, (-)-Enantiomer, ca. 850 mg;
Rt(Enantiomer 2) = 8,613 min; (+)-Enantiomer, ca. 850 mg.

(+)-4-(4-Acetylamino-phenyl)-6,8-dichloro-2,2-dimethyl-1,2,3,4-tetrahydroisochinolinium-iodid (Beispiel 3b);
500 mg (1,43 mmol) des (+)-Enantiomeren von Zwischenprodukt 5 (Enantiomer 2) werden analog der unter Beispiel 3a angegeben Synthesevorschrift mit Methyliodid umgesetzt, wobei 500 mg des gewünschten enantiomerenreinen Ammoniumiodids in Form eines farblosen Feststoffs erhalten werden. (Rt = 1,630 min(Methode B);
MS(ES⁺) = 363,2/365,2).

(+)-4-(4-Acetylamino-phenyl)-6,8-dichloro-2,2-dimethyl-1,2,3,4-tetrahydroisochinolinium-chlorid (Beispiel 3c);
15 g lonenaustauscherharz (Amberlite IRA-93) werden 30 min in 2 N NaOH gerührt und anschließend in eine Säule gepackt. Es wird mit H₂O gespült, bis die pH-Kontrolle neutrale Reaktion anzeigt. Danach wird das Austauscherharz zweimal 15 min in 2 N HCl gerührt, in eine Säule gepackt und wieder mit H₂O gewaschen, bis die pH-Kontrolle neutrale Reaktion anzeigt. Über das so hergestellte Harz (HCl-Form) wird eine Lösung aus 400 mg der Beispielverbindung 3b in 60 ml H₂O gegeben und mit H₂O eluiert. Einengen der Produktfraktionen liefert 303 mg des gewünschten Chloridsalzes als farblosen Feststoff. (Rₜ = 1,990 min(Methode C); MS(ES⁺) = 363,2/365,2).

(-)-4-(4-Acetylamino-phenyl)-6,8-dichloro-2,2-dimethyl-1,2,3,4-tetrahydroisochinolinium, iodid (Beispiel 3d);
Analoges Vorgehen nach der unter Beispielverbindung 3b beschriebenen Methode, ausgehend von dem (-)-Enantiomeren aus Zwischenprodukt 5 (Enantiomer 1) liefert das entsprechende enantiomerenreine Produkt 3d, mit entgegengesetzter Konfiguration zu 3b. (Rt = 1,952 min(Methode A1); MS(ES⁺) = 363,2/365,2).

Beispiel 4: 4-(4-Amino-phenyl)-6,8-dichloro-2,2-dimethyl-1,2,3,4-tetrahydroisochinolinium-chlorid, Hydrochlorid;

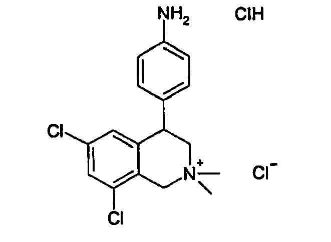

3,5 g (7,13 mmol) 4-(4-Acetylamino-phenyl)-6,8-dichloro-2,2-dimethyl-1,2,3,4-tetrahydro-isochinolinium-iodid (Beispiel 3a) werden in 490 ml 10 %iger HCl und 112 ml Ethanol zwei Stunden zum Rückfluss erhitzt. Nach Abkühlen auf Raumtemperatur wird i. Vak. eingeengt und der Rückstand zwischen H₂O und Essigester verteilt. Die wässrige Phase wird auf ein Volumen von ca, 200 ml eingeengt und nach der in Beispiel 3c beschriebenen Methode einer lonenaustauschchromatographie unterzogen. Einengen des Eluats liefert 2,1 g des gewünschten Hydrochlorids. (Rt = 1,634 min(Methode A1); MS(ES⁺) = 321,0/323,1).

Beispiel 5: 6,8-Dichloro-4-[4-(3-ethyl-ureido)-phenyl]-2,2-dimethyl-1 ,2,3,4-tetrahydroisochinolinium-chlorid;

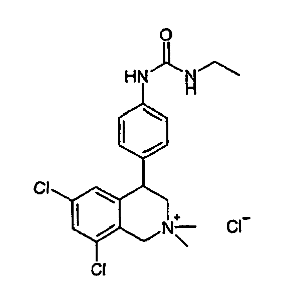

Zwischenprodukt 1: 4-(6,8-Dichloro-2-methyl-1,2,3,4-tetrahydro-isochinolin-4-yl)-phenylamin;
3,0 g (8,59 mmol) N-[4-(6,8-Dichloro-2-methyl-1,2,3,4-tetrahydro-isochinolin-4-yl)-phenyl]-acetamid (Beispiel 3, Zwischenprodukt 5) werden in 100 ml 21 %-iger Natriumethanolatlösung zum Rückfluss erhitzt. Je nach Fortschreiten der Reaktion wird festes Natriumethanolat zugegeben, bis vollständiger Umsatz erreicht ist. Zur Aufarbeitung wird vom Lösungsmittel befreit und der Rückstand in H₂O aufgenommen. Es wird zweimal mit Dichlormethan extrahiert. Die vereinigten organischen Phasen werden mit MgSO₄ getrocknet und eingeengt. Die weitere Reinigung erfolgt durch Chromatographie an Kieselgel mit einem Essigester/Heptan-Gemisch, wobei die Anilinverbindung als gelbes Öl erhalten wird.

Zwischenprodukt 2: 1-[4-(6,8-Dichloro-2-methyl-1,2,3,4-tetrahydro-isochinolin-4-yl)-phenyl]-3-ethyl-harnstoff, Hydrochlorid;
500 mg (1,63 mmol) 4-(6,8-Dichloro-2-methyl-1,2,3,4-tetrahydro-isochinolin-4-yl)-phenylamin werden in 20 ml Toluol vorgelegt und tropfenweise mit einer Lösung aus 284 mg (4,0 mmol) Ethylisocyanat in wenig Toluol versetzt. Nach einer Stunde bei 80 °C werden weitere 180 mg Ethylisocyanat zugegeben und noch ein Stunde bei 80 °C gerührt. Zur Aufarbeitung wird vom Lösung befreit und der Rückstand mit H₂O und Essigester verrieben, abgesaugt und getrocknet, wobei die Titelverbindung als leicht gelblicher Feststoff erhalten wird (Schmp.: 218-220 °C). Der so erhaltene Ethylharnstoff wird in dem Fachmann bekannter Weise in das entsprechende Hydrochlorid überführt.

6,8-Dichloro-4-[4-(3-ethyl-ureido)-phenyl]-2,2-dimethyl-1,2,3,4-tetrahydroisochinolinium-chlorid;
1-[4-(6,8-Dichloro-2-methyl-1,2,3,4-tetrahydro-isochinolin-4-yl)-phenyl]-3-ethyl-hamstoff-Hydrochlorid (300 mg, 0,72 mmol) werden zwischen NaHCO₃ und Essigester verteilt. Der entstandene Niederschlag wird abgesaugt, getrocknet und in 20 ml DMF gelöst. Nach Zugabe von 1,14 g (8,03 mmol) Methyliodid wird drei Stunden bei Raumtemperatur gerührt. Zur Aufarbeitung wird vom Lösungsmittel befreit, der Rückstand in H₂O aufgenommen und mit Essigester extrahiert. Die wässrige Phase wird i. Vak. eingeengt, der Rückstand noch einmal mit Essigester verrieben und abgesaugt. Das so erhaltene quartäre Ammoniumsalz wird nach der in Beispiel 3c beschriebenen Methode einer lonenaustauschchromatographie unterzogen. Einengen des Eluats liefert 90 mg des gewünschten Chlorids. (Rₜ = 2,028 min(Methode A1); MS(ES⁺) = 392,3,0/394,2).

### Pharmakologische Daten:

### Testbeschreibung:

In diesem Test wurde die Erholung des intrazellulären pHs (pHi) nach einer Ansäuerung ermittelt, die bei funktionsfähigem NHE auch unter bicarbonatfreien Bedingungen einsetzt. Dazu wurde der pHi mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHi umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl-Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄ 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀₋Wert der jeweiligen Substanz für die einzelnen NHE-Subtypen berechnet.

### Ergebnisse:

| Beispiel | IC₅₀ [µM], (rNHE3) |
|---|---|
| 1 | 0,23 |
| 2 | 0,90 |
| 3a | 0,67 |
| 3c | 0,43 |
| 4 | 0,03 |

## Patentansprüche

1. Verbindungen der Formel I 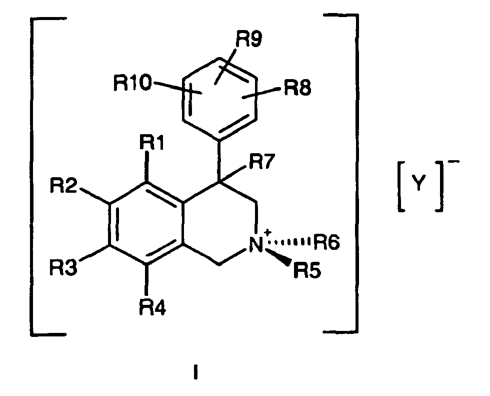 worin bedeuten:
R1, R2, R3 und R4 unabhängig voneinander H, F, Cl, Br, l, CN, NO₂, OH, NH₂, CₐH₂ₐ₊₁ , C_{qq}H_{2qq-1}, OC_{b}H_{2b+1} , COOR50, OCOR50, COR50 oder Oₓ-(CH₂)y-Phenyl;
a und b in den Gruppen CₐH₂ₐ₊₁, und OC_{b}H_{2b+1} unabhängig voneinander 1, 2, 3, 4, 5, 6, 7 oder 8, wobei ein oder mehrere H-Atome durch F-Atome ersetzt sein können;
qq 3, 4, 5, 6, 7 oder 8, wobei ein oder mehrere H-Atome durch F-Atome ersetzt sein können;
R50 H oder C_{c}H_{2c+1};
c 1, 2, 3, 4, 5, 6, 7 oder 8, wobei ein oder mehrere H-Atome durch F-Atome ersetzt sein dürfen,
x Null oder 1;
y Null, 1, 2, 3 oder 4;
wobei der Phenylring in der Gruppe Oₓ-(CH₂)y-Phenyl unsubstituiert ist oder substituiert mit 1 - 3 Substituenten ausgewählt aus der Gruppe bestehend aus F, Cl, Br, CN, NO₂, OH, NH₂ oder C_{d}H_{2d+1};
d 1, 2, 3, oder 4, wobei ein oder mehrere H-Atome durch F-Atome ersetzt sein dürfen;
oder
R1, R2, R3 und R4 unabhängig voneinander Heteroaryl, wobei Null, 1, 2, 3 oder 4 N-Atome, Null oder 1 Sauerstoff-Atom oder Null oder 1 S-Atom als Ringatome enthalten sein können;
oder
A1, R2, R3 und R4 unabhängig voneinander CONR11 R12 oder NR11 R12;
R11 und R12 unabhängig voneinander H, CₑH₂ₑ₊₁, CᵣᵣH₂ᵣᵣ₋₁;
e 1, 2, 3, 4, 5, 6, 7 oder 8,
rr 3, 4, 5, 6, 7, oder 8,
wobei in den Gruppen CₑH₂ₑ₊₁ und CᵣᵣH₂ᵣᵣ₋₁ ein oder mehrere H-Atome durch F-Atome ersetzt sein dürfen und ein oder mehrere CH₂-Gruppen durch O oder NR13 ersetzt sein können;
R13 H oder C_{f}H_{2f+1};
f 1, 2, 3, oder 4, wobei ein oder mehrere H-Atome durch F-Atome ersetzt sein dürfen;
oder
R13 und eine CH₂-Gruppe von R11 oder R12 gemeinsam mit dem N-Atom, an das sie gebunden sind, einen 5- oder 6-gliedrigen Ring;
oder
R11 und R12 gemeinsam mit dem N-Atom, an das sie gebunden sind, einen 5-, 6-oder 7-gliedrigen Ring;
oder
R11 und R12 unabhängig voneinander COR14, CSR14 oder SO₂R14;
R14 C_{g}H_{2g+1};
g 1, 2, 3, 4, 5, 6, 7 oder 8, wobei ein oder mehrere H-Atome durch F-Atome ersetzt sein können und ein oder mehrere CH₂-Gruppen durch O oder NR13 ersetzt sein können,
oder
R1, R2, R3 und R4 unabhängig voneinander -Oₕ-SOⱼ-R15, wobei
h Null oder 1;
j Null, 1 oder 2;
R15 CₖH₂ₖ₊₁, OH, OCₗH₂ₗ₊₁ oder NR17R18;
k 1, 2, 3, 4, 5, 6, 7 oder 8, wobei ein oder mehrere H-Atome durch F-Atome ersetzt sein können;
l 1, 2, 3, 4, 5, 6,7 oder 8, wobei ein oder mehrere H-Atome durch F-Atome ersetzt sein können;
R17 und R18 unabhängig voneinander H oder CₘH₂ₘ₊₁;
m 1, 2, 3, 4, 5, 6, 7 oder 8, wobei in der Gruppe CₘH₂ₘ₊₁ ein oder mehrere H-Atome durch F-Atome ersetzt sein können und ein oder mehrere CH₂₋Gruppen durch O, CO, CS oder NR19 ersetzt sein können;
R19 H oder CₙH₂ₙ₊₁;
n 1, 2, 3 oder 4;
wobei in CₙH₂ₙ₊₁ ein oder mehrere H-Atome durch F-Atome ersetzt sein können;
oder
R17 und R18 gemeinsam mit dem N-Atom, an das sie gebunden sind, einen 5-, 6- oder 7-gliedrigen Ring;
oder
R19 und eine CH₂-Gruppe von R17 oder R18 gemeinsam mit dem N-Atom, an das sie gebunden sind, einen 5- oder 6-gliedrigen Ring;
R5 und R6 unabhängig voneinander CₚH₂ₚ₊₁, CₛₛH₂ₛₛ₋₁, COR20 oder SO₂R20;
p 1, 2, 3, 4, 5, 6, 7 oder 8,
ss 3, 4, 5, 6, 7 oder 8,
R20 C_{q}H_{2q+1};
q 1, 2, 3, 4, 5, 6, 7 oder 8,
wobei in den Gruppen CₚH₂ₚ₊₁, CₛₛH₂ₛₛ₋₁ und C_{q}H_{2q+1} ein oder mehrere H-Atome durch F-Atome ersetzt sein können, und eine oder mehrere CH₂-Gruppen durch O oder NR21 ersetzt sein können;
R21 H oder CᵣH₂ᵣ₊₁;
r 7 , 2, 3 oder 4;
wobei in CᵣH₂ᵣ₊₁ ein oder mehrere H-Atome durch F-Atome ersetzt sein können;
R7 H, F, Cl, Br, I, C_{S}H₂ₛ₊₁, C_{dd}H_{2dd-1}, OH, OCₜH₂ₜ₊₁ oder OCOR22;
s und t unabhängig voneinander 1, 2, 3, 4, 5, 6, 7 oder 8;
dd 3, 4, 5, 6, 7 oder 8, wobei in C_{S}H₂ₛ₊₁, C_{dd}H_{2dd-1} und OCₜH₂ₜ₊₁ ein oder mehrere H-Atome durch F-Atome ersetzt sein können;
R22 CᵤH₂ᵤ₊₁;
u 1, 2, 3 oder 4;
wobei in CᵤH₂ᵤ₊₁ ein oder mehrere H-Atome durch F-Atome ersetzt sein können;
R8, R9 und R10 unabhängig voneinander -Ov-SO_{w}-R23;
v Null oder 1;
w Null, 1 oder 2;
R23 CₙₙH₂ₙₙ₊₁, CₘₘH₂ₘₘ₋₁, OH, OCₚₚH₂ₚₚ₊₁ oder NR25R26;
nn und pp unabhängig voneinander 1, 2, 3, 4, 5, 6, 7 oder 8,
mm 3, 4, 5, 6, 7 oder 8,
wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN oder C_{z}H_{2z+1}, C_{zz}H_{2zz-1};
z 1, 2, 3, 4, 5, 6, 7 oder 8;
zz 3, 4, 5, 6, 7 oder 8, wobei ein oder mehrere H-Atome durch F-Atome ersetzt sein dürfen und in C_{z}H_{2z+1} ein oder mehrere H-Atome durch F-Atome ersetzt sein können und ein oder mehrere CH₂₋Gruppen durch O CO, CS oder NR27 ersetzt sein können;
R27 H oder CₐₐH₂ₐₐ₊₁ ;
aa 1, 2, 3 oder 4;
wobei in CₐₐH₂ₐₐ₊₁ ein oder mehrere H-Atome durch F-Atome ersetzt sein können;
oder
R25 und R26 gemeinsam mit dem N-Atom, an das sie gebunden sind, einen 5-, 6- oder 7-gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
R8, R9 und R10 unabhängig voneinander NR32COR30, NR32CSR30 oder NR32SO_{bb}R30;
R30 H, C_{cc}H_{2cc+1}, C_{yy}H_{2yy-1}, Pyrrolidinyl oder Piperidinyl, in welchen Ringen eine CH₂-Gruppe durch O oder NR33 ersetzt sein kann;
R32 und R33 unabhängig voneinander H oder CₕH₂ₕ₊₁;
bb 2 oder 3;
cc 1, 2, 3, 4, 5, 6, 7 oder 8;
yy 3, 4, 5, 6, 7 oder 8;
h 1, 2, 3, 4, 5, 6, 7 oder 8,
wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂₋Gruppen durch O oder NR31 ersetzt sein können;
R31 H, CₖₖH₂ₖₖ₊₁, COR65;
kk 1, 2, 3, oder 4;
wobei ein oder mehrere H-Atome durch F-Atome ersetzt sein können,
R65 H, CₓₓH₂ₓₓ₊₁;
xx 1, 2, 3 oder 4, wobei ein oder mehrere H-Atome durch F-Atome ersetzt sein dürfen;
oder
R31 zusammen mit einer CH₂-Gruppe von R30 einen 5-, 6- oder 7-gliedrigen Ring bildet;
oder
R30 ein 5- oder 6-gliedriges Heteroaryl mit 1, 2, 3 oder 4 N-Atomen, Null oder 1 S-Atomen und Null oder 1 O-Atomen,
das unsubstituiert ist oder substituiert mit bis zu drei Substituenten ausgewählt aus der Gruppe bestehend aus F, Cl, Br, l, CₒₒH₂ₒₒ₊₁, NR70R71;
R70 und R71 unabhängig voneinander H, CᵤᵤH₂ᵤᵤ₊₁ und COR72;
R72 H, CᵥᵥH₂ᵥᵥ₊₁;
oo, uu und w unabhängig voneinander 1, 2, 3, 4, 5, 6, 7 oder 8;
wobei in den Gruppen CₒₒH₂ₒₒ₊₁, CᵤᵤH₂ᵤᵤ₊₁ oder C_{w}H_{2w+1} ein oder mehrere H-Atome durch F-Atome ersetzt sein dürfen;
oder
R8, R9 und R10 unabhängig voneinander H, F, Cl, Br, l, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42,
ee und ff unabhängig voneinander 1, 2, 3, 4, 5, 6, 7 oder 8;
ww 3, 4, 5, 6, 7 oder 8;
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₜₜ₊₁;
tt 1, 2, 3, 4, 5, 6, 7 oder 8;
wobei in der Gruppe CₜₜH₂ₜₜ₊₁ ein oder mehrere H-Atome durch F-Atome ersetzt sein dürfen und eine oder mehrere CH₂-Gruppen durch O- oder NR44 ersetzt sein können;
R44 H oder C_{gg}H_{2gg+1};
gg 1, 2, 3, 4, 5, 6, 7 oder 8;
wobei in der Gruppe C_{gg}H_{2gg+1} ein oder mehrere H-Atome durch F-Atome ersetzt sein können,
oder
R40 und R41 mit dem N-Atom, an das sie gebunden sind, einen 5- oder 6-gliedrigen Ring;
R42 H oder CₕₕH₂ₕₕ₊₁,
hh 1, 2, 3, 4, 5, 6, 7 oder 8;
wobei in der Gruppe CₕₕH₂ₕₕ₊₁ ein oder mehrere H-Atome durch F-Atome ersetzt sein können;
Y Fluor, Chlor, Brom, lod, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate;
wobei R2 nicht gleich H ist

2. Verbindungen der Formel I nach Anspruch 1, worin bedeuten:
R1, R2, R3 und R4 unabhängig voneinander H, F, Cl, Br, l, CN, NO₂, OH, NH₂, CₐH₂ₐ₊₁, Cycloalkyl mit 3, 4, 5 oder 6 C-Atomen, OC_{b}H_{2b+1}, COOR50;
a und b unabhängig voneinander 1, 2, 3 oder 4, wobei ein oder mehrere H-Atome durch F-Atome ersetzt sein können;
R50 H oder C_{c}H_{2c+1};
c 1, 2, 3 oder 4, wobei ein oder mehrere H-Atome durch F-Atome ersetzt sein dürfen;
oder
R1, R2, R3 und R4 unabhängig voneinander 5- oder 6-gliedriges Heteroaryl, ausgewählt aus der Gruppe bestehend aus Imidazolyl, Pyrazolyl, Pyrrolyl, Triazolyl, Tetrazolyl, Thiazolyl und Oxazolyl;
oder
R1, R2, R3 und R4 unabhängig voneinander CONR11 R12 oder NR11 R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen
oder
R11 und R12 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl;
oder
R11 und R12 gemeinsam mit dem N-Atom, an das sie gebunden sind, einen Pyrrolidin-, Piperidin-, N-Methylpiperazin-, Piperazin- oder Morpholinring;
oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein;
oder
R1, R2, R3 und R4 unabhängig voneinander OSO₃H, SO₃H, SO₂R₁₅, wobei
R15 CₖH₂ₖ₊₁, OCₗH₂ₗ₊₁ oder NR17R18;
k 1, 2, 3 oder 4, wobei ein oder mehrere H-Atome durch F-Atome ersetzt sein können;
l 1, 2, 3 oder 4, wobei ein oder mehrere H-Atome durch F-Atome ersetzt sein können;
R17 und R18 unabhängig voneinander H, CₘH₂ₘ₊₁ oder CₘH₂ₘ₊₁, in welchem die an den Stickstoff gebundene erste CH₂-Gruppe durch CO und die zweite CH₂-Gruppe durch NR19 ersetzt ist;
m 1, 2, 3, 4 oder 5, wobei in der Gruppe CₘH₂ₘ₊₁ ein oder mehrere H-Atome durch F-Atome ersetzt sein können;
R19 H oder CₙH₂ₙ₊₁;
n 1, 2, 3 oder 4;
wobei in CₙH₂ₙ₊₁ ein oder mehrere H-Atome durch F-Atome ersetzt sein können;
oder
R17 und R18 gemeinsam mit dem N-Atom, an das sie gebunden sind, einen 5- oder, 6-gliedrigen Ring;
R5 und R6 unabhängig voneinander CₚH₂ₚ₊₁;
p 1, 2, 3 oder 4;
wobei in CₚH₂ₚ₊₁ ein oder mehrere H-Atome durch F-Atome ersetzt sein können;
R7 H, CₛH₂ₛ₊₁, OCₜH₂ₜ₊₁ oder OCOR22;
s und t unabhängig voneinander 1, 2, 3 oder 4;
wobei in CₛH₂ₛ₊₁ und OCₜH₂ₜ₊₁ ein oder mehrere H-Atome durch F-Atome ersetzt sein können;
R22 CᵤH₂ᵤ₊₁;
u 1, 2, 3 oder 4;
wobei in CᵤH₂ᵤ₊₁ ein oder mehrere H-Atome durch F-Atome ersetzt sein können;
R8, R9 und R10 unabhängig voneinander OSO₃H, SO₃H oder SO₂R23;
R23 CₙₙH₂ₙₙ₊₁, CₘₘH₂ₘₘ₋₁, OCₚₚH₂ₚₚ₊₁ oder NR25R26;
nn und pp unabhängig voneinander 1, 2, 3, 4 oder 5,
mm 3, 4, 5 oder 6,
wobei in CₙₙH₂ₙₙ₊₁, CₘₘH₂ₘₘ₋₁ und OCₚₚH₂ₚₚ₊₁ ein oder mehrere H-Atome durch F-Atome ersetzt sein können;
R25 und R26 unabhängig voneinander H, CN, C_{z}H_{2z+1} oder C_{z}H_{2z+1} , in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein;
R27 H oder CₐₐH₂ₐₐ₊₁;
aa 1, 2,3 oder 4;
wobei in CₐₐH₂ₐₐ₊₁ ein oder mehrere H-Atome durch F-Atome ersetzt sein können;
oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
R8, R9 und R10 unabhängig voneinander NR32COR30, NR32CSR30 oder NR32SO₂R30;
R30 H, OH, C_{cc}H_{2cc+1}, C_{yy}H_{2yy-1}; Pyrrolidinyl oder Piperidinyl, in welchen Ringen eine CH₂-Gruppe durch O oder NR33 ersetzt sein kann;
R32 und R33 unabhängig von einander H oder CₕH_{2h+1;}
cc 1, 2, 3, 4, 5 oder 6;
yy 3, 4, 5 oder 6;
h 1, 2, 3 oder 4;
wobei in CₕH₂ₕ₊₁ ein oder mehrere H-Atome durch F-Atome ersetzt sein dürfen und in den Gruppen C_{cc}H_{2cc+1} und C_{yy}H_{2yy-1} ein oder mehrere H-Atome durch F-Atome ersetzt sein können und eine oder mehrere CH₂₋Gruppen durch O oder NR31 ersetzt sein können;
R31 H, CₖₖH₂ₖₖ₊₁ oder COR65;
kk 1, 2, 3, oder 4;
wobei ein oder mehrere H-Atome durch F-Atome ersetzt sein können,
R65 H, CₓₓH_{2xx+1;}
xx 1, 2, 3 oder 4, wobei ein oder mehrere H-Atome durch F-Atome ersetzt sein dürfen;
oder
R31 zusammen mit einer CH₂-Gruppe von R30 und dem N-Atom, an das sie gemeinsam gebunden sind einen 5- oder 6-gliedrigen Ring bilden;
oder
R30 einen 5- oder 6-gliedrigen Heteroaromaten ausgewählt aus der Gruppe bestehend aus Pyridyl, Imidazolyl, Pyrazolyl, Pyrrolyl, Triazolyl, Tetrazolyl, Thienyl, Thiazolyl und Oxazolyl,
die unsubstituiert ist oder substituiert mit bis zu drei Substituenten ausgewählt aus der Gruppe bestehend aus F, Cl, Br, l, CₒₒH₂ₒₒ₊₁, NR70R71,
R70 und R71 unabhängig voneinander H, CᵤᵤH₂ᵤᵤ₊₁ oder COR72,;
R72 H, CᵥᵥH_{2vv+1;}
oo, uu und w unabhängig voneinander 1, 2, 3 oder 4; wobei in den Gruppen CₒₒH₂ₒₒ₊₁, CᵤᵤH₂ᵤᵤ₊₁ oder CᵥᵥH₂ᵥᵥ₊₁ ein oder mehrere H-Atome durch F-Atome ersetzt sein dürfen;
oder
R8, R9 und R10 unabhängig voneinander H, F, Cl, Br, l, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 oder OCOR42;
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{ff+1} ein oder mehrere H-Atome durch F-Atome ersetzt sein können;
R40 und R41 H oder CₜₜH₂ₜₜ₊₁;
tt 1, 2, 3, 4, 5, 6, 7 oder 8;
wobei in der Gruppe CₜₜH₂ₜₜ₊₁ ein oder mehrere H-Atome durch F-Atome ersetzt sein dürfen;
oder
R40 und R41 unabhängig voneinander auszuwählen Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl;
oder
R40 und R41 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
R42 H oder CₕₕH₂ₕₕ₊₁;
hh 1, 2, 3 oder 4;
wobei in der Gruppe CₕₕH₂ₕₕ₊₁ ein oder mehrere H-Atome durch F-Atome ersetzt sein können;
Y Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

3. Verbindungen der Formel I nach Ansprüchen 1 oder 2, worin bedeuten:
R1, R2, R3 und R4 unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
R1, R2, R3 und R4 unabhängig voneinander NR11 R12;
R11 und R12 unabhängig voneinander H, CₑH₂ₑ₊₁, CᵣᵣH₂ᵣᵣ₋₁;
e 1, 2, 3 oder 4,
rr 3, 4, 5 oder 6,
wobei in den Gruppen CₑH₂ₑ₊₁ und CᵣᵣH₂ᵣᵣ₋₁ ein oder mehrere H-Atome durch F-Atome ersetzt sein dürfen;
oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
oder
R11 und R12 unabhängig voneinander COR14, CSR14, CONHR14, CSNHR14 oder SO₂R14;
R14 C_{g}H_{2g+1};
g 1, 2, 3 oder 4, wobei ein oder mehrere H-Atome durch F-Atome ersetzt sein können;
oder
R1, R2, R3 und R4 unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können;
oder
R17 und R18 gemeinsam mit dem N-Atom, an das sie gebunden sind, einen 5- oder, 6-gliedrigen Ring;
R5 und R6 unabhängig voneinander Methyl oder Trifluormethyl;
R7 H;
R8, R9 und R10 unabhängig voneinander OSO₃H, SO₃H oder SO₂R23;
R23 CₙₙH₂ₙₙ₊₁ oder NR25R26;
nn 1,2,3,4 oder 5,
wobei in CₙₙH₂ₙₙ₊₁ ein oder mehrere H-Atome durch F-Atome ersetzt sein können;
R25 und R26 unabhängig voneinander H, CN oder C_{Z}H_{2z+1}, oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{z}H_{2z+1} ein oder mehrere H-Atome durch F-Atome ersetzt sein können;
R27 H oder CₐₐH₂ₐₐ₊₁ ;
aa 1, 2, 3 oder 4;
wobei in CₐₐH₂ₐₐ₊₁ ein oder mehrere H-Atome durch F-Atome ersetzt sein können;
oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
R8, R9 und R10 unabhängig voneinander NR32COR30, NR32CSR30 oder NR32SO₂R30;
R30 H, OH, C_{cc}H_{2cc+1}. C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5 oder 6;
yy 3, 4, 5 oder 6;
wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch O oder NR31 ersetzt sein können;
R31 H, Methyl, Ethyl, CF₃, CH₂CF₃, Acetyl oder Propionyl;
oder
R31 zusammen mit einer CH₂-Gruppe von R30 und dem N-Atom, an das sie gemeinsam gebunden sind, einen 5- oder 6-gliedrigen Ring bilden;
oder
R30 Pyridyl, Imidazolyl, Pyrazolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
R8, R9 und R10 unabhängig voneinander H, F, Cl, OH, NH₂, CₑₑH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₜₜ₊₁;
tt 1, 2, 3 oder 4;
wobei in der Gruppe CₜₜH₂ₜₜ₊₁ ein oder mehrere H-Atome durch F-Atome ersetzt sein dürfen;
oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl;
oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂ₕₕ₊₁;
hh 1, 2, 3 oder 4;
wobei in der Gruppe CₕₕH₂ₕₕ₊₁ ein oder mehrere H-Atome durch F-Atome ersetzt sein können;
Y Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

4. Verbindungen der Formel I nach Ansprüchen 1 - 3, worin bedeuten:
R1 und R3 H;
R2 und R4 unabhängig voneinander H, F, Cl, Br, OH, NH₂, CₐH₂ₐ₊₁, Cycloalkyl mit 3, 4, 5 oder 6 C-Atomen, OC_{b}H_{2b+1};
a und b in den Gruppen CₐH₂ₐ₊₁ und OC_{b}H_{2b+1} unabhängig voneinander 1, 2, 3 oder 4, wobei ein oder mehrere H-Atome durch F-Atome ersetzt sein können;
oder
R2 und R4 unabhängig voneinander NR11 R12;
R11 und R12 unabhängig voneinander H, CₑH₂ₑ₊₁,
e 1, 2, 3 oder 4,
wobei in CₑH₂ₑ₊₁ ein oder mehrere H-Atome durch F-Atome ersetzt sein dürfen;
oder
R11 und R12 gemeinsam mit dem N-Atom, an das sie gebunden sind, einen Ring bilden, ausgewählt aus der Gruppe bestehend aus Pyrrolidin, Piperidin, N-Methylpiperazin, Piperazin und Morpholin;
oder
R11 und R12 unabhängig voneinander COR14, CSR14, CONHR14, CSNHR14 oder SO₂R14;
R14 C_{g}H_{2g+1} ;
g 1, 2, 3 oder 4, wobei ein oder mehrere H-Atome durch F-Atome ersetzt sein können;
oder
R2 und R4 unabhängig voneinander OSO₃H, SO₃H, SO₂R15;
R15 CₖH₂ₖ₊₁ oder NR17R18;
k 1, 2, 3 oder 4, wobei ein oder mehrere H-Atome durch F-Atome ersetzt sein können;
R17 und R18 unabhängig voneinander H oder CₘH₂ₘ₊₁;
m 1, 2, 3, 4 oder 5, wobei in der Gruppe CₘH₂ₘ₊₁ ein oder mehrere H-Atome durch F-Atome ersetzt sein können;
oder
R17 und R18 gemeinsam mit dem N-Atom, an das sie gebunden sind, einen 5- oder, 6-gliedrigen Ring;
R5 und R6 unabhängig voneinander Methyl oder Trifluormethyl;
R7 H;
R8, R9 und R10 unabhängig voneinander OSO₃H, SO₃H oder SO₂R23;
R23 CₙₙH₂ₙₙ₊₁ oder NR25R26;
nn 1, 2, 3, 4 oder 5,
wobei in CₙₙH₂ₙₙ₊₁ ein oder mehrere H-Atome durch F-Atome ersetzt sein können;
R25 und R26 unabhängig voneinander H, CN oder C_{z}H_{2z+1}, oder C_{z}H_{2z+1}, in welchem die an den Stickstoff gebundene erste CH₂-Gruppe durch CO oder CS und die zweite CH₂- durch NR27 ersetzt ist;
z 1, 2, 3, 4, 5 oder 6;
wobei in C_{Z}H_{2z+1} ein oder mehrere H-Atome durch F-Atome ersetzt sein können;
R27 H oder CₐₐH₂ₐₐ₊₁ ;
aa 1, 2, 3 oder 4;
wobei in CₐₐH₂ₐₐ₊₁ ein oder mehrere H-Atome durch F-Atome ersetzt sein können;
oder
R25 und R26 gemeinsam mit dem N-Atom, an das sie gebunden sind, einen 5- oder 6- gliedrigen Ring,
oder
R27 und eine CH₂-Gruppe von R25 oder R26 gemeinsam mit dem N-Atom, an das sie gebunden sind, einen 5- oder 6-gliedrigen Ring;
oder
R8, R9 und R10 unabhängig voneinander NR32COR30, NR32CSR30 oder NR32SO₂R30;
R₃₀ H, OH, C_{cc}H_{2cc+1}. C_{yy}H_{2yy-1}, Pyrrolidinyl oder Piperidinyl, in welchen Ringen eine CH₂-Gruppe durch O oder NR33 ersetzt sein kann;
R32 und R33 H, Methyl oder CF₃;
cc 1, 2, 3, 4, 5 oder 6;
yy 3, 4, 5 oder 6;
wobei in den Gruppen C_{cc}H_{2cc+1} und C_{yy}H_{2yy-1} ein oder mehrere H-Atome durch F-Atome ersetzt sein können und eine oder mehrere CH₂-Gruppen durch O oder NR31 ersetzt sein können;
R31 H, Methyl, Ethyl, CF₃, CH₂CF₃, Acetyl oder Propionyl;
oder
R31 zusammen mit einer CH₂-Gruppe von R30 und dem N-Atom, an das sie gemeinsam gebunden sind, einen 5- oder 6-gliedrigen Ring bilden;
oder
R30 Pyridyl, Imidazolyl, Pyrazolyl, Triazolyl, Tetrazolyl, Thiazolyl oder Oxazolyl, die unsubstituiert sind oder substituiert mit maximal 3 Substituenten ausgewählt aus der Gruppe bestehend aus F, Cl, Methyl, Ethyl, Trifluormethyl, NH₂, NHAcetyl;
oder
R8, R9 und R10 unabhängig voneinander H, F, Cl, OH, NH₂, CeeH₂ₑₑ₊₁, C_{ww}H_{2ww-1}, OC_{ff}H_{2ff+1}, NR40R41, CONR40R41, COOR42 oder OCOR42,
ee und ff unabhängig voneinander 1, 2, 3 oder 4;
ww 3, 4, 5 oder 6,
wobei in den Gruppen CₑₑH₂ₑₑ₊₁, C_{ww}H_{2ww-1} und OC_{ff}H_{2ff+1} ein oder mehrere H-Atome durch F-Atome ersetzt sein können;
R40 und R41 H oder CₜₜH₂ₙ₊₁;
tt 1, 2, 3 oder 4;
wobei in der Gruppe CₜₜH₂ₜₜ₊₁ ein oder mehrere H-Atome durch F-Atome ersetzt sein dürfen;
oder
R40 und R41 unabhängig voneinander Hydroxyethyl, N,N-Dimethylaminoethyl, N,N-Diethylaminoethyl, Pyrrolidinoethyl, N-Methylpiperazinoethyl, Piperazinoethyl, Morpholinoethyl oder Piperidinoethyl;
oder
R40 und R41 gemeinsam mit dem N-Atom, an das sie gebunden sind, einen Pyrrolidin-, Piperidin-, N-Methylpiperazin-, Piperazin- oder Morpholinring;
R42 H oder CₕₕH₂hh+1;
hh 1, 2, 3 oder 4;
wobei in der Gruppe CₕₕH₂ₕₕ₊₁ ein oder mehrere H-Atome durch F-Atome ersetzt sein können;
Y Fluor, Chlor, Brom, Hydroxy, sowie alle anionischen Formen pharmakologisch verträglicher Mono-, Di- oder Tricarbonsäuren oder Sulfonsäuren;
sowie deren pharmazeutisch verträgliche Salze und Trifluoracetate.

5. Verbindung nach einem der Ansprüche 1 - 4, **dadurch gekennzeichnet, dass** sie sind:
a. 6,8-Dichloro-2,2-dimethyl-4-phenyl-1,2,3,4-tetrahydro-isochinolinium-trifluoracetat;
b. 6,8-Dichloro-2,2-dimethyl-4-(4-sulfamoyl-phenyl)-1,2,3,4-tetrahydro-isochinolinium-trifluoracetat;
c. 4-(4-Acetylamino-phenyl)-6,8-dichloro-2,2-dimethyl-1,2,3,4-tetrahydroisochinolinium-iodid;
d. (+)-4-(4-Acetylamino-phenyl)-6,8-dichloro-2,2-dimethyl-1,2,3,4-tetrahydroisochinolinium-iodid;
e. (-)-4-(4-Acetylamino-phenyl)-6,8-dichloro-2,2-dimethyl-1,2,3,4-tetrahydroisochinolinium-iodid;
f. (+)-4-(4-Acetylamino-phenyl)-6,8-dichloro-2,2-dimethyl-1,2,3,4-tetrahydroisochinolinium-chlorid;
g. 4-(4-Amino-phenyl)-6,8-dichloro-2,2-dimethyl-1,2,3,4-tetrahydro-isochinolinium-chlorid; Hydrochlorid;
h. 6,8-Dichloro-4-[4-(3-ethyl-ureido)-phenyl]-2,2-dimethyl-1,2,3,4-tetrahydroisochinolinium-chlorid;
sowie deren pharmazeutisch verträgliche Salze.

6. Verwendung einer Verbindung I nach Anspruch 1 zur Herstellung eines Medikaments zur Behandlung oder Prophylaxe von Störungen des Atemantriebs.

7. Verwendung einer Verbindung I nach Anspruch 1 zur Herstellung eines Medikaments zur Behandlung oder Prophylaxe von Atemstörungen, insbesondere Schlaf-bedingten Atemstörungen wie Schlafapnoen.

8. Verwendung einer Verbindung I nach Anspruch 1 zur Herstellung eines Medikaments zur Behandlung oder Prophylaxe des Schnarchens.

9. Verwendung einer Verbindung 1 nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

10. Verwendung einer Verbindung I nach Anspruch 1 zur Herstellung eines Medikaments zur Behandlung oder Prophylaxe von Störungen der Darmfunktion.

11. Verwendung einer Verbindung I nach Anspruch 1 zur Herstellung eines Medikaments zur Behandlung oder Prophylaxe von Störungen der Gallenfunktion.

12. Verwendung einer Verbindung 1 nach Anspruch 1 zur Herstellung eines Medikaments zur Behandlung oder Prophylaxe von ischämischen Zuständen des peripheren und zentralen Nervensystems und des Schlaganfalls.

13. Verwendung einer Verbindung I nach Anspruch 1 zur Herstellung eines Medikaments zur Behandlung oder Prophylaxe von ischämischen Zuständen peripherer Organe und Gliedmaßen.

14. Verwendung einer Verbindung I nach Anspruch 1 zur Herstellung eines Medikaments zur Behandlung von Schockzuständen.

15. Verwendung einer Verbindung I nach Anspruch 1 zur Herstellung eines Medikaments zu Einsatz bei chirurgischen Operationen und Organtransplantationen.

16. Verwendung einer Verbindung I nach Anspruch 1 zur Konservierung und Lagerung von Transplantaten für chirurgische Maßnahmen.

17. Verwendung einer Verbindung I nach Anspruch 1 zur Herstellung eines Medikaments zur Behandlung von Krankheiten, bei denen die Zellproliferation eine primäre oder sekundäre Ursache darstellt.

18. Verwendung einer Verbindung 1 nach Anspruch 1 zur Herstellung eines Medikaments zur Behandlung oder Prophylaxe von Störungen des Fettstoffwechsels.

19. Verwendung einer Verbindung I nach Anspruch 1 zur Herstellung eines Medikaments zur Behandlung oder Prophylaxe des Befalls durch Ektoparasiten.

20. Heilmittel, enthaltend eine wirksame Menge einer Verbindung I nach Anspruch 1.

## Claims

1. A compound of the formula I 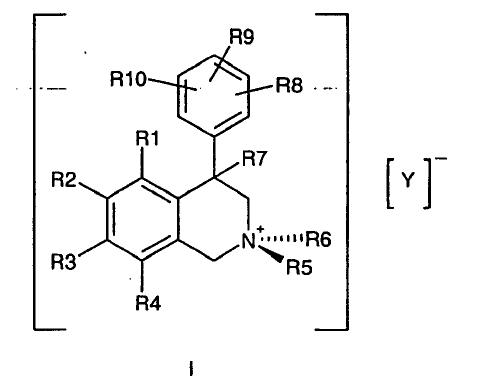 in which the meanings are:
R1, R2, R3 and R4 independently of one another H, F, Cl, Br, I, CN, NO₂, OH, NH₂, CₐH₂ₐ₊₁, C_{qq}H_{2qq-1}, OC_{b}H_{2b+1}, COOR50, OCOR50, COR50 or Oₓ-(CH₂)_{y}-phenyl;
a and b in the groups CₐH₂ₐ₊₁ and OC_{b}H_{2b+1} independently of one another 1, 2, 3, 4, 5, 6, 7 or 8, it being possible for one or more H atoms to be replaced by F atoms;
qq 3, 4, 5, 6, 7 or 8, it being possible for one or more H atoms to be replaced by F atoms;
R50 H or C_{c}H_{2c+1};
c 1, 2, 3, 4, 5, 6, 7 or 8, it being possible for one or more H atoms to be replaced by F atoms,
x zero or 1;
y zero, 1, 2, 3 or 4;
where the phenyl ring in the group Oₓ-(CH₂)_{y-}phenyl is unsubstituted or substituted by 1-3 substituents selected from the group consisting of F, Cl, Br, CN, NO₂, OH, NH₂ or C_{d}H_{2d+1;}
d 1, 2, 3, or 4, it being possible for one or more H atoms to be replaced by F atoms;
or
R1, R2, R3 and R4 independently of one another heteroaryl, it being possible for zero, 1, 2, 3 or 4 N atoms, zero or 1 oxygen atom or zero or 1 S atom to be present as ring atoms;
or
R 1, R2, R3 and R4 independently of one another CONR11 R12 or NR11 R12;
R11 and R12 independently of one another H, CₑH₂ₑ₊₁, CᵣᵣH₂ᵣᵣ₋₁;
e 1, 2, 3, 4, 5, 6, 7 or 8,
rr 3, 4, 5, 6, 7, or 8,
it being possible for one or more H atoms in the groups CₑH₂ₑ₊₁ and CᵣᵣH₂ᵣᵣ₋₁ to be replaced by F atoms and for one or more CH₂ groups to be replaced by O or NR13;
R13 H or C_{f}H_{2f+1};
f 1, 2, 3, or 4, it being possible for one or more H atoms to be replaced by F atoms;
or
R13 and a CH₂ group of R11 or R12 together with the N atom to which they are bonded a 5- or 6-membered ring;
or
R11 and R12 together with the N atom to which they are bonded a 5-, 6- or 7-membered ring;
or
R11 and R12 independently of one another COR14, CSR14 or SO₂R14;
R14 C_{g}H_{2g+1};
g 1, 2, 3, 4, 5, 6, 7 or 8, it being possible for one or more H atoms to be replaced by F atoms, and it being possible for one or more CH₂ groups to be replaced by O or NR13,
or
R1, R2, R3 and R4 independently of one another -Oₕ-SOⱼ-R15, with
h zero or 1;
j zero, 1 or 2;
R15 CₖH₂ₖ₊₁, OH, OCₗH₂ₗ₊₁ or NR17R18;
k 1, 2, 3, 4, 5, 6, 7 or 8, it being possible for one or more H atoms to be replaced by F atoms;
l 1, 2, 3, 4, 5, 6, 7 or 8, it being possible for one or more H atoms to be replaced by F atoms;
R17 and R18 independently of one another H or CₘH₂ₘ₊₁;
m 1, 2, 3, 4, 5, 6, 7 or 8, it being possible for one or more H atoms in the group CₘH₂ₘ₊₁ to be replaced by F atoms and for one or more CH₂ groups to be replaced by O, CO, CS or NR19;
R19 H or CₙH₂ₙ₊₁;
n 1, 2, 3 or 4; it being possible for one or more H atoms in CₙH₂ₙ₊₁ to be replaced by F atoms;
or
R17 and R18 together with the N atom to which they are bonded a 5-, 6- or 7-membered ring;
or
R19 and a CH₂ group or R17 or R18 together with the N atom to which they are bonded a 5- or 6-membered ring;
R5 and R6 independently of one another CₚH₂ₚ₊₁, CₛₛH₂ₛₛ₋₁, COR20 or SO₂R20;
p 1, 2, 3, 4, 5, 6, 7 or 8,
ss 3, 4, 5, 6, 7 or 8,
R20 C_{q}H_{2q+1};
q 1, 2, 3, 4, 5, 6, 7 or 8,
it being possible for one or more H atoms in the groups CpH₂ₚ₊₁, CₛₛH₂ₛₛ₋₁ and CqH_{2q+1} to be replaced by F atoms and for one or more CH₂ groups to be replaced by O or NR21;
R21 H or CᵣH₂ᵣ₊₁;
r 1,2,3 or 4;
it being possible for one or more H atoms in CᵣH₂ᵣ₊₁ to be replaced by F atoms;
R7 H, F, Cl, Br, l, CₛH₂ₛ₊₁, C_{dd}H_{2dd-1}, OH, OCₜH₂ₜ₊₁ or OCOR22;
s and t independently of one another 1, 2, 3, 4, 5, 6, 7 or 8;
dd 3, 4, 5, 6, 7 or 8, it being possible for one or more H atoms in CₛH₂ₛ₊₁, C_{dd}H_{2dd-1} and OCₜH₂ₜ₊₁ to be replaced by F atoms;
R22 CᵤH₂ᵤ₊₁;
u 1, 2, 3 or 4;
it being possible for one or more H atoms in CᵤH₂ᵤ₊₁ to be replaced by F atoms;
R8, R9 and R10 independently of one another -Oᵥ-SO_{w}-R23;
v zero or 1;
w zero, 1 or 2;
R23 CₙₙH₂ₙₙ₊₁, CₘₘH₂ₘₘ₋₁, OH, OCₚₚH₂ₚₚ₊₁ or NR25R26;
nn and pp independently of one another 1, 2, 3, 4, 5, 6, 7 or 8,
mm 3, 4, 5, 6, 7 or 8,
it being possible for one or more H atoms in CₙₙH₂ₙₙ₊₁, CₘₘH₂ₘₘ₋₁ and OCₚₚH₂ₚₚ₊₁ to be replaced by F atoms;
R25 and R26 independently of one another H, CN or C_{z}H_{2z+1}, C_{zz}H_{2zz-1};
z 1, 2, 3, 4, 5, 6, 7 or 8;
zz 3, 4, 5, 6, 7 or 8, it being possible for one or more H atoms to be replaced by F atoms and, in C_{z}H_{2z+1}, it being possible for one or more H atoms to be replaced by F atoms and it being possible for one or more CH₂ groups to be replaced by O, CO, CS or NR27;
R27 H or CₐₐH₂ₐₐ₊₁;
aa 1, 2, 3 or 4;
it being possible for one or more H atoms in CₐₐH₂ₐₐ₊₁ to be replaced by F atoms;
or
R25 and R26 together with the N atom to which they are bonded a 5-, 6- or 7-membered ring,
or
R27 and a CH₂ group of R25 or R26 together with the N atom to which they are bonded a 5- or 6-membered ring;
or
R8, R9 and R10 independently of one another NR32COR30, NR32CSR30 or NR32SO_{bb}R30;
R30 H, C_{cc}H_{2cc+1} C_{yy}H_{2yy-1}, pyrrolidinyl or piperidinyl, in which rings a CH₂ group may be replaced by O or NR33;
R32 and R33 independently of one another H or CₕH₂ₕ₊₁;
bb 2 or 3;
cc 1, 2, 3, 4, 5, 6, 7 or 8;
yy 3, 4, 5, 6, 7 or 8;
h 1, 2, 3, 4, 5, 6, 7 or 8,
it being possible for one or more H atoms in CₕH₂ₕ₊₁ to be replaced by F atoms, and it being possible for one or more H atoms in the groups C_{cc}H_{2cc+1} and C_{yy}H_{2yy-1} to be replaced by F atoms and for one or more CH₂ groups to be replaced by O or NR31;
R31 H, CₖₖH₂ₖₖ₊₁, COR65;
kk 1, 2, 3, or 4;
it being possible for one or more H atoms to be replaced by F atoms,
R65 H, CₓₓH₂ₓₓ₊₁;
xx 1, 2, 3 or 4, it being possible for one or more H atoms to be replaced by F atoms;
or
R31 forms together with a CH₂ group of R30 a 5-, 6- or 7-membered ring;
or
R30 a 5- or 6-membered heteroaryl with 1; 2, 3 or 4 N atoms, zero or 1 S atoms and zero or 1 O atoms,
which is unsubstituted or substituted by up to three substituents selected from the group consisting of F, Cl, Br, l, CₒₒH₂ₒₒ₊₁, NR70R71;
R70 and R71 independently of one another H, CᵤᵤH₂ᵤᵤ₊₁ and COR72;
R72 H, CᵥᵥH₂ᵥᵥ₊₁;
oo, uu and w independently-of one another 1, 2, 3, 4, 5, 6, 7 or 8;
it being possible for one or more H atoms in the groups CₒₒH₂ₒₒ₊₁, CᵤᵤH₂ᵤᵤ₊₁ or CᵥᵥH₂ᵥᵥ₊₁ to be replaced by F atoms;
or
R8, R9 and R10 independently of one another H, F, Cl, Br, I, NO₂, CN, OH, NH₂, CₑₑH₂ₑₑ₊₁, C_{ww}H_{2ww-1}; OC_{ff}H₂₊₁, NR40R41, CONR40R41, COOR42, COR42 or OCOR42,
ee and ff independently of one another 1, 2, 3, 4, 5, 6, 7 or 8;
ww 3, 4, 5, 6, 7 or 8;
it being possible for one or more H atoms in the groups CₑₑH₂ₑₑ₊₁, C_{ww}H_{2ww-1} and OC_{ff}H_{2ff+1} to be replaced by F atoms;
R40 and R41 H or CₜₜH₂ₜₜ₊₁;
tt 1, 2, 3, 4, 5, 6, 7 or 8;
it being possible for one or more H atoms in the group CₜₜH₂ₜₜ₊₁ to be replaced by F atoms and for one or more CH₂ groups to be replaced by O or NR44;
R44 H or C_{gg}H_{2gg+1};
gg 1, 2, 3, 4, 5, 6, 7 or 8;
it being possible for one or more H atoms in the group C_{gg}H_{2gg+1} to be replaced by F atoms,
or
R40 and R41 with the N atom to which they are bonded a 5- or 6-membered ring;
R42 H or CₕₕH_{2hh+1;}
hh 1,2,3,4,5,6,7 or 8;
it being possible for one or more H atoms in the group CₕₕH₂ₕₕ₊₁ to be replaced by-F atoms;
Y fluorine, chlorine, bromine, iodine, hydroxyl and all anionic forms of pharmacologically acceptable mono-, di- or tricarboxylic acids or sulfonic acids;
and the pharmaceutically acceptable salts and trifluoroacetates thereof; wherein R2 is not equal to H.

2. A compound of the formula I as claimed in claim 1, in which the meanings are
R1, R2, R3 and R4 independently of one another H, F, Cl, Br, I, CN, NO₂, OH, NH₂, CₐH₂ₐ₊₁, cycloalkyl with 3, 4, 5 or 6 C atoms, OC_{b}H_{2b+1}, COOR50;
a and b independently of one another 1, 2, 3 or 4, it being possible for one or more H atoms to be replaced by F atoms;
R50 H or C_{c}H_{2c+1};
c 1, 2, 3 or 4, it being possible for one or more H atoms to be replaced by F atoms;
or
R1, R2, R3 and R4 independently of one another 5- or 6-membered heteroaryl selected from the group consisting of imidazolyl, pyrazolyl, pyrrolyl, triazolyl, tetrazolyl, thiazolyl and oxazolyl;
or
R1, R2, R3 and R4 independently of one another CONR11 R12 or NR11 R12;
R11 and R12 independently of one another H, CₑH₂ₑ₊₁, CᵣᵣH₂ᵣᵣ₋₁;
e 1, 2, 3 or 4,
rr 3, 4, 5 or 6,
it being possible for one or more H atoms in the groups CₑH₂ₑ₊₁ and CᵣᵣH₂ᵣᵣ₋₁ to be replaced by F atoms
or
R11 and R12 independently of one another hydroxyethyl, N,N-dimethylaminoethyl, N,N-diethylaminoethyl, pyrrolidinoethyl, N-methylpiperazinoethyl, piperazinoethyl, morpholinoethyl or piperidinoethyl;
or
R11 and R12 together with the N atom to which they are bonded a pyrrolidine, piperidine, N-methylpiperazine, piperazine or morpholine ring;
or
R11 and R12 independently of one another COR14, CSR14, CONHR14, CSNHR14 or SO₂R14;
R14 C_{g}H_{2g+1};
g 1, 2, 3 or 4, it being possible for one or more H atoms to be replaced by F atoms;
or
R1, R2, R3 and R4 independently of one another OSO₃H, SO₃H, SO₂R₁₅, with
R15 CₖH₂ₖ₊₁, OCₗH₂ₗ₊₁ or NR17R18;
k 1, 2, 3 or 4, it being possible for one or more H atoms to be replaced by F atoms;
l 1, 2, 3 or 4, it being possible for one or more H atoms to be replaced by F atoms;
R17 and R18 independently of one another H, CₘH₂ₘ₊₁ or CₘH₂ₘ₊₁, in which the first CH₂ group bonded to the nitrogen is replaced by CO and the second CH₂ group is replaced by NR19;
m 1, 2, 3, 4 or 5, it being possible for one or more H atoms in the group CₘH₂ₘ₊₁ to be replaced by F atoms;
R19 H or CₙH₂ₙ₊₁;
n 1, 2, 3 or 4;
it being possible for one or more H atoms in CₙH₂ₙ₊₁ to be replaced by F atoms;
or
R17 and R18 together with the N atom to which they are bonded a 5- or 6-membered ring;
R5 and R6 independently of one another CₚH₂ₚ₊₁;
p 1, 2, 3 or 4;
it being possible for one or more H atoms in CₚH₂ₚ₊₁ to be replaced by F atoms;
R7 H, C_{S}H₂ₛ₊₁, OCₜH₂ₜ₊₁ or OCOR22;
s and t independently of one another 1, 2, 3 or 4; it being possible for one or more H atoms in C_{S}H₂ₛ₊₁ and OCₜH₂ₜ₊₁ to be replaced by F atoms;
R22 CᵤH₂ᵤ₊₁ ;
u 1,2,3or4;
it being possible for one or more H atoms in CᵤH₂ᵤ₊₁ to be replaced by F atoms;
R8, R9 and R10 independently of one another OSO₃H, SO₃H or SO₂R23;
R23 CₙₙH₂ₙₙ₊₁, CₘₘH₂ₘₘ₋₁, OCₚₚH₂ₚₚ₊₁ or NR25R26;
nn and pp independently of one another 1, 2, 3, 4 or 5,
mm 3, 4, 5 or 6,
it being possible for one or more H atoms in CₙₙH₂ₙₙ₊₁ , CₘₘH₂ₘₘ₋₁ and OCₚₚH₂ₚₚ₊₁ to be replaced by F atoms;
R25 and R26 independently of one another H, CN, C_{z}H_{2z+1} or C_{z}H_{2z+1}, in which the first CH₂ group bonded to the nitrogen is replaced by CO or CS and the second CH₂ is replaced by NR27;
z 1, 2, 3, 4, 5 or 6;
it being possible for one or more H atoms in C_{Z}H_{2z+1} to be replaced by F atoms;
R27 H or CₐₐH₂ₐₐ₊₁;
aa 1, 2, 3 or 4;
it being possible for one or more H atoms in CₐₐH₂ₐₐ₊₁ to be replaced by F atoms;
or
R25 and R26 together with the N atom to which they are bonded a 5- or 6-membered ring;
or
R27 and a CH₂ group of R25 or R26 together with the N atom to which they are bonded a 5- or 6-membered ring;
or
R8, R9 and R10 independently of one another NR32COR30, NR32CSR30 or NR32SO₂R30;
R30 H, OH, C_{cc}H_{2cc+1}, C_{yy}H_{2yy-1;} pyrrolidinyl or piperidinyl, in which rings a CH₂ group may be replaced by O or NR33;
R32 and R33 independently of one another H or CₕH₂ₕ₊₁;
cc 1, 2, 3, 4, 5 or 6;
yy 3, 4, 5 or 6;
h 1, 2, 3 or 4;
it being possible for one or more H atoms in CₕH₂ₕ₊₁ to be replaced by F atoms, and it being possible for one or more H atoms in the groups C_{cc}H_{2cc+1} and C_{yy}H_{2yy-1} to be replaced by F atoms and for one or more CH₂ groups to be replaced by O or NR31;
R3 1 H, CₖₖH₂ₖₖ₊₁ or COR65;
kk 1, 2, 3, or 4;
it being possible for one or more H atoms to be replaced by F atoms,
R65 H, CₓₓH₂ₓₓ₊₁;
xx 1, 2, 3 or 4, it being possible for one or more H atoms to be replaced by F atoms;
or
R31 together with a CH₂ group of R30 and the N atom to which they are jointly bonded form a 5- or 6-membered ring;
or
R30 a 5- or 6-membered heteroaromatic system selected from the group consisting of pyridyl, imidazolyl, pyrazolyl, pyrrolyl, triazolyl, tetrazolyl, thienyl, thiazolyl and oxazolyl, which is unsubstituted or substituted by up to three substituents selected from the group consisting of F, Cl, Br, I, CₒₒH₂ₒₒ₊₁, NR70R71,
R70 and R71 independently of one another H, CᵤᵤH₂ᵤᵤ₊₁ or COR72;
R72 H, CᵥᵥH₂ᵥᵥ₊₁ ;
oo, uu and vv independently of one another 1, 2, 3 or 4;
it being possible for one or more H atoms in the groups CₒₒH₂ₒₒ₊₁, CᵤᵤH₂ᵤᵤ₊₁ or CᵥᵥH₂ᵥᵥ₊₁ to be replaced by F atoms;
or
R8, R9 and R10 independently of one another H, F, Cl, Br, I, NO₂, CN, OH, NH₂, CₑₑH₂ₑₑ₊₁, C_{ww}H_{2ww-1}, OC_{ff} H_{2ff+1}, NR40R41, CONR40R41, COOR42, COR42 or OCOR42;
ee and ff independently of one another 1, 2, 3 or 4;
ww 3, 4, 5 or 6,
it being possible for one or more H atoms in the groups CₑₑH₂ₑₑ₊₁, C_{ww}H_{2ww-1} and OC_{ff}H_{2ff+1} to be replaced by F atoms;
R40 and R41 H or CₜₜH₂ₜₜ₊₁;
tt 1, 2, 3, 4, 5, 6, 7 or 8;
it being possible for one or more H atoms in the group CₜₜH₂ₜₜ₊₁ to be replaced by F atoms;
or
R40 and R41 to be selected independently of one another hydroxyethyl, N,N-dimethylaminoethyl, N,N-diethylaminoethyl, pyrrolidinoethyl, N-methylpiperazinoethyl, piperazinoethyl, morpholinoethyl or piperidinoethyl;
or
R40 and R41 together with the N atom to which they are bonded form a ring selected from the group consisting of pyrrolidine, piperidine, N-methylpiperazine, piperazine and morpholine;
R42 H or CₕₕH₂ₕₕ₊₁ ;
hh 1, 2, 3 or 4;
it being possible for one or more H atoms in the group CₕₕH₂ₕₕ₊₁ to be replaced by F atoms;
Y fluorine, chlorine, bromine, hydroxyl and all anionic forms of pharmacologically acceptable mono-, di- or tricarboxylic acids or sulfonic acids;
and the pharmaceutically acceptable salts and trifluoroacetates thereof.

3. A compound of the formula I as claimed in claim 1 or 2, in which the meanings are:
R1, R2, R3 and R4 independently of one another H, F, Cl, Br, OH, NH₂, CₐH₂ₐ₊₁, cycloalkyl with 3, 4, 5 or 6 C atoms, OC_{b}H_{2b+1};
a and b in the groups CₐH₂ₐ₊₁ and OC_{b}H_{2b+1} independently of one another 1, 2, 3 or 4, it being possible for one or more H atoms to be replaced by F atoms;
or
R1, R2, R3 and R4 independently of one another NR11 R12;
R11 and R12 independently of one another H, CₑH₂ₑ₊₁, CᵣᵣH₂ᵣᵣ₋₁;
e 1, 2, 3 or 4,
rr 3, 4, 5 or 6,
it being possible for one or more H atoms in the groups CₑH₂ₑ₊₁ and CᵣᵣH₂ᵣᵣ₋₁ to be replaced by F atoms;
or
R11 and R12 together with the N atom to which they are bonded form a ring selected from the group consisting of pyrrolidine, piperidine, N-methylpiperazine, piperazine and morpholine;
or
R11 and R12 independently of one another COR14, CSR14, CONHR14, CSNHR14 or SO₂R₁₄;
R14 C_{g}H_{2g+1};
g 1, 2, 3 or 4, it being possible for one or more H atoms to be replaced by F atoms;
or
R1, R2, R3 and R4 independently of one another OSO₃H, SO₃H, SO₂R15;
R15 CₖH₂ₖ₊₁ or NR17R18;
k 1, 2, 3 or 4, it being possible for one or more H atoms to be replaced by F atoms;
R17 and R18 independently of one another H or CₘH₂ₘ₊₁:
m 1, 2, 3, 4 or 5, it being possible for one or more H atoms in the group CₘH₂ₘ₊₁ to be replaced by F atoms;
or
R17 and R18 together with the N atom to which they are bonded a 5- or 6-membered ring;
R5 and R6 independently of one another methyl or trifluoromethyl;
R7 H;
R8, R9 and R10 independently of one another OSO₃H, SO₃H or SO₂R23;
R23 CₙₙH₂ₙₙ₊₁ or NR25R26;
nn 1, 2, 3, 4 or 5,
it being possible for one or more H atoms in CₙₙH₂ₙₙ₊₁ to be replaced by F atoms;
R25 and R26 independently of one another H, CN or C_{z}H_{2z+1}, or C_{z}H_{2z+1} in which the first CH₂ group bonded to the nitrogen is replaced by CO or CS and the second CH₂ is replaced by NR27;
z 1, 2, 3, 4, 5 or 6;
it being possible for one or more H atoms in C_{z}H_{2z+1} to be replaced by F atoms;
R27 H or CₐₐH₂ₐₐ₊₁;
aa 1, 2, 3 or 4;
it being possible for one or more H atoms in CₐₐH₂ₐₐ₊₁ to be replaced by F atoms;
or
R25 and R26 together with the N atom to which they are bonded a 5- or 6-membered ring,
or
R27 and a CH₂ group of R25 or R26 together with the N atom to which they are bonded a 5- or 6-membered ring;
or
R8, R9 and R10 independently of one another NR32COR30, NR32CSR30 or NR32SO₂R30;
R30 H, OH, C_{cc}H_{2cc+1}, C_{yy}H_{2YY-1}, pyrrolidinyl or piperidinyl, in which rings a CH₂ group may be replaced by O or NR33;
R32 and R33 H, methyl or CF₃;
cc 1, 2, 3, 4, 5 or 6;
yy 3, 4, 5 or 6;
it being possible for one or more H atoms in the groups C_{cc}H_{2cc+1} and C_{yy}H_{2yy-1} to be replaced by F atoms and for one or more CH₂ groups to be replaced by O or NR31;
R31 H, methyl, ethyl, CF₃, CH₂CF₃, acetyl or propionyl; or
R31 together with a CH₂ group of R30 and the N atom to which they are jointly bonded form a 5- or 6-membered ring;
or
R30 pyridyl, imidazolyl, pyrazolyl, triazolyl, tetrazolyl, thiazolyl or oxazolyl, which are unsubstituted or substituted by a maximum of 3 substituents selected from the group consisting of F, Cl, methyl, ethyl, trifluoromethyl, NH₂, NHacetyl;
or
R8, R9 and R10 independently of one another H, F, Cl, OH, NH₂, CₑₑH₂ₑₑ₊₁, C_{ww}H_{2ww-1}, OC_{ff}H_{2ff+1}, NR40R41, CONR40R41, COOR42 or OCOR42,
ee and ff independently of one another 1, 2, 3 or 4;
ww 3, 4, 5 or 6,
it being possible for one or more H atoms in the groups CₑₑH₂ₑₑ₊₁, C_{ww}H_{2ww-1} and OC_{ff}H_{2ff+1} to be replaced by F atoms;
R40 and R41 H or CₜₜH₂ₜₜ₊₁;
tt 1, 2, 3 or 4;
it being possible for one or more H atoms in the group CₜₜH₂ₜₜ₊₁ to be replaced by F atoms;
or
R40 and R41 independently of one another hydroxyethyl, N,N-dimethylaminoethyl, N,N-diethylaminoethyl, pyrrolidinoethyl, N-methylpiperazinoethyl, piperazinoethyl, morpholinoethyl or piperidinoethyl;
or
R40 and R41 together with the N atom to which they are bonded a pyrrolidine, piperidine, N-methylpiperazine, piperazine or morpholine ring;
R42 H or CₕₕH₂ₕₕ₊₁;
hh 1, 2, 3 or 4;
it being possible for one or more H atoms in the group ChₕH₂ₕₕ₊₁ to be replaced by F atoms;
Y fluorine, chlorine, bromine, hydroxyl and all anionic forms of pharmacologically acceptable mono-, di- or tricarboxylic acids or sulfonic acids;
and the pharmaceutically acceptable salts and trifluoroacetates thereof.

4. A compound of the formula I as claimed in claims 1-3, in which the meanings are:
R1 and R3 H;
R2 and R4 independently of one another H, F, Cl, Br, OH, NH₂, CₐH₂ₐ₊₁, cycloalkyl with 3, 4, 5 or 6 C atoms, OC_{b}H_{2b+1};
a and b in the groups CₐH₂ₐ₊₁ and OC_{b}H_{2b+1} independently of one another 1, 2, 3 or 4, it being possible for one or more H atoms to be replaced by F atoms;
or
R2 and R4 independently of one another NR11 R12;
R11 and R12 independently of one another H, CₑH₂ₑ₊₁,
e 1, 2, 3 or 4,
it being possible for one or more H atoms in CₑH₂ₑ₊₁ to be replaced by F atoms;
or
R11 and R12 together with the N atom to which they are bonded form a ring selected from the group consisting of pyrrolidine, piperidine, N-methylpiperazine, piperazine and morpholine;
or
R11 and R12 independently of one another COR14, CSR14, CONHR14, CSNHR14 or SO₂R14;
R14 C_{g}H_{2g+1};
g 1, 2, 3 or 4, it being possible for one or more H atoms to be replaced by F atoms;
or
R2 and R4 independently of one another OSO₃H, SO₃H, SO₂R15;
R15 CₖH₂ₖ₊₁ or NR17R18;
k 1, 2, 3 or 4, it being possible for one or more H atoms to be replaced by F atoms;
R17 and R18 independently of one another H or CₘH₂ₘ₊₁;
m 1, 2, 3, 4 or 5, it being possible for one or more H atoms in the group CₘH₂ₘ₊₁ to be replaced by F atoms;
or
R17 and R18 together with the N atom to which they are bonded a 5- or 6-membered ring;
R5 and R6 independently of one another methyl or trifluoromethyl;
R7 H;
R8, R9 and R10 independently of one another OSO₃H, SO₃H or SO₂R23;
R23 CₙₙH₂ₙₙ₊₁ or NR25R26;
nn 1,2,3,4or5,
it being possible for one or more H atoms in CnₙH₂ₙₙ₊₁ to be replaced by F atoms;
R25 and R26 independently of one another H, CN or C_{z}H_{2z+1}, or C_{z}H_{2z+1} in which the first CH₂ group bonded to the nitrogen is replaced by CO or CS and the second CH₂ is replaced by NR27;
z 1, 2, 3, 4, 5 or 6;
it being possible for one or more H atoms in C_{z}H_{2z+1} to be replaced by F atoms;
R27 H or CₐₐH_{2aa+1;}
aa 1, 2, 3 or 4;
it being possible for one or more H atoms in CₐₐH₂ₐₐ₊₁ to be replaced by F atoms;
or
R25 and R26 together with the N atom to which they are bonded a 5- or 6-membered ring,
or
R27 and a CH₂ group of R25 or R26 together with the N atom to which they are bonded a 5- or 6-membered ring;
or
R8, R9 and R10 independently of one another NR32COR30, NR32CSR30 or NR32SO₂R30;
R30 H, OH, C_{cc}H_{2cc+1}, C_{yy}H_{2yy-1}, pyrrolidinyl or piperidinyl, in which rings a CH₂ group may be replaced by O or NR33;
R32 and R33 H, methyl or CF₃;
cc 1, 2, 3, 4, 5 or 6;
yy 3, 4, 5 or 6;
it being possible for one or more H atoms in the groups C_{cc}H_{2cc+1},- and C_{yy}H_{2yy-1} to be replaced by F atoms and for one or more CH₂ groups to be replaced by O or NR31;
R31 H, methyl, ethyl, CF₃, CH₂CF₃, acetyl or propionyl;
or
R31 together with a CH₂ group of R30 and the N atom to which they are jointly bonded form a 5- or 6-membered ring;
or
R30 pyridyl, imidazolyl, pyrazolyl, triazolyl, tetrazolyl, thiazolyl or oxazolyl, which are unsubstituted or substituted by a maximum of 3 substituents selected from the group consisting of F, Cl, methyl, ethyl, trifluoromethyl, NH₂, NHacetyl;
or
R8, R9 and R10 independently of one another H, F, Cl, OH, NH₂, CₑₑH₂ₑₑ₊₁, C_{ww}H_{2ww-1}, OC_{ff}H_{2ff+1}, NR40R41, CONR40R41, COOR42 or OCOR42,
ee and ff independently of one another 1, 2, 3 or 4;
ww 3, 4, 5 or 6,
it being possible for one or more H atoms in the groups CₑₑH₂ₑₑ₊₁, C_{ww}H_{2ww-1} and OC_{ff}H_{2ff+1} to be replaced by F atoms;
R40 and R41 H or CₜₜH₂ₜₜ₊₁ ;
tt 1, 2, 3 or 4;
it being possible for one or more H atoms in the group CₜₜH₂ₜₜ₊₁ to be replaced by F atoms;
or
R40 and R41 independently of one another hydroxyethyl, N,N-dimethylaminoethyl, N,N-diethylaminoethyl, pyrrolidinoethyl, N-methylpiperazinoethyl, piperazinoethyl, morpholinoethyl or piperidinoethyl;
or
R40 and R41 together with the N atom to which they are bonded a pyrrolidine, piperidine, N-methylpiperazine, piperazine or morpholine ring;
R42 H or CₕₕH₂ₕₕ₊₁;
hh 1, 2, 3 or 4;
it being possible for one or more H atoms in the group CₕₕH₂ₕₕ₊₁ to be replaced by F atoms;
Y fluorine, chlorine, bromine, hydroxyl and all anionic forms of pharmacologically acceptable mono-, di- or tricarboxylic acids or sulfonic acids;
and the pharmaceutically acceptable salts and trifluoroacetates thereof.

5. A compound as claimed in any of claims 1-4, **characterized in that** it is:
a. 6,8-dichloro-2,2-dimethyl-4-phenyl-1,2,3,4-tetrahydroisoquinolinium trifluoroacetate;
b. 6,8-dichloro-2,2-dimethyl-4-(4-sulfamoylphenyl)-1,2,3,4-tetrahydroisoquinolinium trifluoroacetate;
c. 4-(4-acetylaminophenyl)-6,8-dichloro-2,2-dimethyl-1,2,3,4-tetrahydroisoquinolinium iodide;
d. (+)-4-(4-acetylaminophenyl)-6,8-dichloro-2,2-dimethyl-1,2,3,4-tetrahydro-isoquinolinium iodide;
e. (-)-4-(4-acetylaminophenyl)-6,8-dichloro-2,2-dimethyl-1,2,3,4-tetrahydro-isoquinolinium iodide;
f. (+)-4-(4-acetylaminophenyl)-6,8-dichloro-2,2-dimethyl-1,2,3,4-tetrahydro-isoquinolinium chloride;
g. 4-(4-aminophenyl)-6,8-dichloro-2,2-dimethyl-1,2,3,4-tetrahydroisoquinolinium chloride; hydrochloride;
h. 6,8-dichloro-4-[4-(3-ethylureido)phenyl]-2,2-dimethyl-1,2,3,4-tetrahydroisoquinolinium chloride;
and the pharmaceutically acceptable salts thereof.

6. The use of a compound I as claimed in claim 1 for producing a medicament for the treatment or prophylaxis of disorders of respiratory drive.

7. The use of a compound I as claimed in claim 1 for producing a medicament for the treatment or prophylaxis of respiratory disorders, in particular sleep-related respiratory disorders such as sleep apneas.

8. The use of a compound I as claimed in claim 1 for producing a medicament for the treatment or prophylaxis of snoring.

9. The use of a compound I as claimed in claim 1 for producing a medicament for the treatment or prophylaxis of acute and chronic renal disorders, particularly of acute renal failure and of chronic renal failure.

10. The use of a compound I as claimed in claim 1 for producing a medicament for the treatment or prophylaxis of disorders of intestinal function.

11. The use of a compound I as claimed in claim 1 for producing a medicament for the treatment or prophylaxis of disorders of biliary function.

12. The use of a compound I as claimed in claim 1 for producing a medicament for the treatment or prophylaxis of ischemic states of the peripheral and central nervous system and of stroke.

13. The use of a compound I as claimed in claim 1 for producing a medicament for the treatment or prophylaxis of ischemic states of the peripheral organs and limbs.

14. The use of a compound I as claimed in claim 1 for producing a medicament for the treatment of states of shock.

15. The use of a compound I as claimed in claim 1 for producing a medicament for use in surgical operations and organ transplantations.

16. The use of a compound I as claimed in claim 1 for the preservation and storage of transplants for surgical procedures.

17. The use of a compound I as claimed in claim 1 for producing a medicament for the treatment of disorders in which cell proliferation represents a primary or secondary cause.

18. The use of a compound I as claimed in claim 1 for producing a medicament for the treatment or prophylaxis of disorders of lipid metabolism.

19. The use of a compound I as claimed in claim 1 for producing a medicament for the treatment or prophylaxis of infestation by ectoparasites.

20. A medicine comprising an effective amount of a compound I as claimed in claim 1.

## Revendications

1. Composés de la formule I : 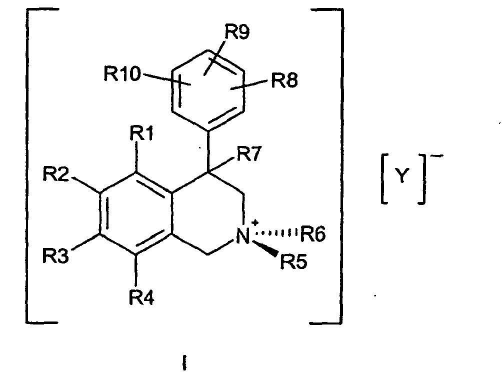 dans laquelle
R1, R2, R3 et R4 représentent indépendamment l'un de l'autre H, F, Cl, Br, 1, CN, NO₂, OH, NH₂, CₐH₂ₐ₊₁,
C_{qq}H_{2qq-1}, OC_{b}H_{2b+1},COOR50, OCOR50, COR50 ou Oₓ-(CH₂)_{y}-phényle,
a et b représentent dans les groupes CₐH₂ₐ₊₁, et OC_{b}H_{2b+1}, indépendamment l'un de l'autre, 1, 2, 3, 4, 5, 6, 7 ou 8, un ou plusieurs atomes de H pouvant être remplacés par des atomes de F,
qq vaut 3, 4, 5, 6, 7 ou 8, un ou plusieurs atomes de H pouvant être remplacés par des atomes de F,
R50 représente H ou C_{c}H_{2c+1},
c valant 1, 2, 3, 4, 5, 6, 7 ou 8, un ou plusieurs atomes de H pouvant être remplacés par des atomes de F,
x vaut zéro ou 1,
y vaut zéro, 1, 2, 3 ou 4,
le noyau de phényle dans le groupe Oₓ-(CH₂)_{y}-phényle étant non substitué ou substitué par 1 à 3 substituants choisis parmi le groupe constitué de F, CI, Br, CN, NO₂, OH, NH₂ ou C_{d}H_{2d+1},
d vaut 1, 2, 3 ou 4, un ou plusieurs atomes de H pouvant être remplacés par des atomes de F,
ou
R1, R2, R3 et R4 représentent indépendamment l'un de l'autre un groupe hétéroaryle, dans lequel zéro, 1, 2, 3 ou 4 atomes de N, zéro ou 1 atome d'oxygène ou zéro ou 1 atome de S peuvent être contenus comme atomes du noyau,
ou
R1, R2, R3 et R4 représentent indépendamment l'un de l'autre CONR11 R12 ou NR11R12,
R11 et R12 représentent indépendamment l'un de l'autre H, CₑH₂ₑ₊₁, CᵣᵣH₂ᵣᵣ₋₁,
e vaut 1, 2, 3, 4, 5, 6, 7 ou 8,
rr vaut 3, 4, 5, 6, 7 ou 8, '
un ou plusieurs atomes de H pouvant être remplacés par des atomes de F et un ou plusieurs groupes CH₂ pouvant être remplacés par O ou NR13 dans les groupes CₑH₂ₑ₊₁ et CᵣᵣH₂ᵣᵣ₋₁
R13 représente H ou C_{f}H_{2f+1},
f vaut 1, 2, 3 ou 4, un ou plusieurs atomes de H pouvant être remplacés par des atomes de F,
ou
R13 et un groupe CH₂ forment conjointement avec l'atome de N auquel ils sont liés un noyau pentagonal ou hexagonal,
ou
R11 et R12 forment conjointement avec l'atome de N auquel ils sont liés un noyau pentagonal, hexagonal ou heptagonal,
ou
R11 et R12 représentent indépendamment l'un de l'autre COR14, CSR14 ou SO₂R14,
R14 représente C_{g}H_{2g+1},
g vaut 1, 2, 3, 4, 5, 6, 7 ou 8, un ou plusieurs atomes de H pouvant être remplacés par des atomes de F et un ou plusieurs groupes CH₂ pouvant être remplacés par O ou NR13,
ou
R1, R2, R3 et R4 représentent indépendamment l'un de l'autre -On-SOj-R15, où
h vaut zéro ou 1,
j vaut zéro, 1 ou 2,
R15 représente CₖH₂ₖ₊₁, OH, OCₗH₂ₗ₊₁ ou NR17R18,
k vaut 1, 2, 3, 4, 5, 6, 7 ou 8, un ou plusieurs atomes de H pouvant être remplacés par des atomes de F,
l vaut 1, 2, 3, 4, 5, 6, 7 ou 8, un ou plusieurs atomes de H pouvant être remplacés par des atomes de F,
R17 et R18 représentent indépendamment l'un de l'autre H ou CₘH₂ₘ₊₁,
m vaut 1, 2, 3, 4, 5, 6, 7 ou 8, un ou plusieurs atomes de H pouvant être remplacés par des atomes de F et un ou plusieurs groupes CH₂ pouvant être remplacés par O, CO, CS ou NR19 dans le groupe CₘH₂ₘ₊₁,
R19 représente H ou CₙH₂ₙ₊₁,
n vaut 1, 2, 3 ou 4,
un ou plusieurs atomes de H pouvant être remplacés par des atomes de F dans CₙH₂ₙ₊₁,
ou
R17 et R18 forment conjointement avec l'atome de N, auquel ils sont liés, un noyau pentagonal, hexagonal ou heptagonal,
ou
R19 et un groupe CH₂ de R17 ou R18 forment conjointement avec l'atome de N auquel ils sont liés un noyau pentagonal ou hexagonal,
R5 et R6 représentent indépendamment l'un de l'autre CₚH₂ₚ₊₁, CₛₛH₂ₛₛ₋₁, COR20 ou SO₂R20,
p vaut 1, 2, 3, 4, 5, 6, 7 ou 8,
ss vaut 3, 4, 5, 6, 7 ou 8,
R20 représente C_{q}H_{2q+1},
q vaut 1, 2, 3, 4, 5, 6, 7 ou 8,
un ou plusieurs atomes de H pouvant être remplacés par des atomes de F et un ou plusieurs groupes CH2 pouvant être remplacés par O ou NR21 dans les groupes CₚH₂ₚ₊₁, CₛₛH₂ₛₛ₋₁ et C_{q}H_{2q+1},
R21 représente H ou CᵣH_{2r+1,}
r vaut1,2,3ou4,
un ou plusieurs atomes de H pouvant être remplacés par des atomes de F dans CᵣH₂ᵣ₊₁,
R7 représente H, F, Cl, Br, l, CₛH₂ₛ₊₁, C_{dd}H_{2dd-1}, OH, OCₜH₂ₜ₊₁ ou OCOR22,
s et t représentent indépendamment l'un de l'autre 1, 2, 3, 4, 5, 6, 7 ou 8,
dd vaut 3, 4, 5, 6, 7 ou 8, un ou plusieurs atomes de H pouvant être remplacés par des atomes de F dans CₛH₂ₛ₊₁, C_{dd}H_{2dd-1} et OCₜH₂ₜ₊₁,
R22 représente CᵤH₂ᵤ₊₁,
u vaut 1, 2, 3 ou 4,
un ou plusieurs atomes de H pouvant être remplacés par des atomes de F dans CᵤH₂ᵤ₊₁,
R8, R9 et R10 représentent indépendamment l'un de l'autre -Oᵥ-SO_{w}-R23,
v vaut zéro ou 1,
w vaut zéro, 1 ou 2,
R23 représente CₙₙH₂ₙₙ₊₁, CₘₘH₂ₘₘ₋₁, OH, OCₚₚH₂ₚₚ₊₁ ou NR25R26,
nn et pp représentent indépendamment l'un de l'autre 1, 2, 3, 4, 5, 6, 7 ou 8,
mm vaut 3, 4, 5, 6, 7 ou 8,
un ou plusieurs atomes de H pouvant être remplacés par des atomes de F dans CₙₙH₂ₙₙ₊₁, CₘₘH₂ₘₘ₋₁ et OCₚₚH₂ₚₚ₊₁,
R25 et R26
représentent indépendamment l'un de l'autre H, CN ou C_{z}H_{2z+1}, C_{zz}H_{2zz-1}
z vaut 1, 2, 3, 4, 5, 6, 7 ou 8,
zz vaut 3, 4, 5, 6, 7 ou 8, un ou plusieurs atomes de H pouvant être remplacés par des atomes de F et, dans C_{z}H_{2z+1}, un ou plusieurs atomes de H pouvant être remplacés par des atomes de F et un ou plusieurs groupes CH₂ pouvant être remplacés par O, CO, CS ou NR27,
R27 représente H ou CₐₐH₂ₐₐ₊₁,
aa vaut 1, 2, 3 ou 4, un ou plusieurs atomes de H pouvant être remplacés par des atomes de F dans CₐₐH₂ₐₐ₊₁,
ou
R25 et R26 forment conjointement avec l'atome de N auquel ils sont liés un noyau pentagonal, hexagonal ou heptagonal,
ou
R27 et un groupe CH₂ de R25 ou R26 forment conjointement avec l'atome de N auquel ils sont liés un noyau pentagonal ou hexagonal,
ou
R8, R9 et R10 représentent indépendamment l'un de l'autre NR32COR30, NR32CSR30 ou NR32SO_{bb}R30,
R30 représente H, C_{cc}H_{2cc+1}, C_{yy}H_{2yy-1} , pyrrolidinyle ou pipéridinyle, noyaux dans lesquels un groupe CH₂ peut être remplacé par O ou NR33,
R32 et R33 représentent indépendamment l'un de l'autre H ou CₕH₂ₕ₊₁,
bb vaut 2 ou 3,
cc vaut 1, 2, 3, 4, 5, 6, 7 ou 8,
yy vaut 3, 4, 5, 6, 7 ou 8,
h vaut 1, 2, 3, 4, 5, 6, 7 ou 8,
où, dans CₕH₂ₕ₊₁, un ou plusieurs atomes de H peuvent être remplacés par des atomes de F et, dans les groupes C_{cc}H_{2cc+1} et C_{yy}H_{2yy-1}, un ou plusieurs atomes de H peuvent être remplacés par des atomes de F et un ou plusieurs groupes CH₂ peuvent être remplacés par O ou NR31,
R31 représente H, CₖₖH₂ₖₖ₊₁, COR65,
kk vaut 1, 2, 3 ou 4, un ou plusieurs atomes de H peuvent être remplacés par des atomes de F,
R65 représente H, CₓₓH₂ₓₓ₊₁,
xx vaut 1, 2, 3 ou 4, un ou plusieurs atomes de H pouvant être remplacés par des atomes de F,
ou
R31 forme conjointement avec un groupe CH₂ de R30 un noyau pentagonal, hexagonal ou heptagonal,
ou
R30 représente un hétéroaryle pentagonal ou hexagonal comportant 1, 2, 3 ou 4 atomes de N, zéro ou 1 atome de S et zéro ou 1 atome de O, qui est non substitué ou substitué avec jusqu'à trois substituants choisis parmi le groupe constitué de F, CI, Br, 1, CₒₒH₂ₒₒ₊₁, NR70R71,
R70 et R71 représentent indépendamment l'un de l'autre H, CᵤᵤH₂ᵤᵤ₊₁ et COR72,
R72 représente H, CᵥᵥH₂ᵥᵥ₊₁,
oo, uu et vv valent indépendamment l'un de l'autre 1, 2, 3, 4, 5, 6, 7 ou 8,
un ou plusieurs atomes de H pouvant être remplacés par des atomes de F dans les groupes CₒₒH₂ₒₒ₊₁, CᵤᵤH₂ᵤᵤ₊₁ ou CᵥᵥH₂ᵥᵥ₊₁,
ou
R8, R9 et R10 représentent indépendamment l'un de l'autre H, F, Cl, Br, I, NO₂, CN, OH, NH₂, CₑₑH₂ₑₑ₊₁, C_{ww}H_{2ww-1}, OC_{ff}H_{2ff+1}, NR40R41, CONR40R41, COOR42, COR42 ou OCOR42,
ee et ff valent indépendamment l'un de l'autre 1, 2, 3, 4, 5, 6, 7 ou 8,
ww vaut 3, 4, 5, 6, 7 ou 8,
un ou plusieurs atomes de H pouvant être remplacés par des atomes de F dans les groupes CₑₑH₂ee+₁, C_{ww}H_{2ww-1} et OC_{ff}H_{2ff+1},
R40 et R41 représentent H ou CₜₜH₂ₜₜ₊₁,
tt vaut 1, 2, 3, 4, 5, 6, 7 ou 8,
un ou plusieurs atomes de H pouvant être remplacés par des atomes de F et un ou plusieurs groupes CH2 pouvant être remplacés par O ou NR44 dans le groupe CₜₜH₂ₜₜ₊₁,
R44 représente H ou C_{gg}H_{2gg+1},
gg vaut 1, 2, 3, 4, 5, 6, 7 ou 8,
un ou plusieurs atomes de H pouvant être remplacés par des atomes de F dans le groupe C_{gg}H_{2gg+1},
ou
R40 et R41 forment conjointement avec l'atome de N auquel ils sont liés un noyau pentagonal ou hexagonal,
R42 représente H ou CₕₕH₂ₕₕ₊₁,
hh vaut 1, 2, 3, 4, 5, 6, 7 ou 8,
un ou plusieurs atomes de H pouvant être remplacés par des atomes de F dans le groupe CₕₕH₂ₕₕ₊₁ ,
Y représente du fluor, du chlore, du brome, de l'iode, un hydroxy, ainsi que toutes les formes anioniques d'acides monocarboxyliques, dicarboxyliques ou tricarboxyliques ou sulfoniques pharmacologiquement compatibles,
ainsi que leurs sels pharmaceutiquement compatibles et trifluoroacétates,
R² n'étant pas égal à H.

2. Composés de la formule I suivant la revendication 1, dans lesquels
R1, R2, R3 et R4 représentent indépendamment l'un de l'autre H, F, CI, Br, I, CN, NO₂, OH, NH₂, CₐH₂ₐ₊₁, un groupe cycloalkyle comportant 3, 4, 5 ou 6 atomes de C, OC_{b}H_{2b+1}, COOR50,
a et b représentent indépendamment l'un de l'autre 1, 2, 3 ou 4, un ou plusieurs atomes de H pouvant être remplacés par des atomes de F,
R50 représente H ou, C_{c}H_{2c+1},
c vaut 1, 2, 3 ou 4, un ou plusieurs des atomes de H pouvant être remplacés par des atomes de F,
ou
R1, R2, R3 et R4 représentent, indépendamment l'un de l'autre, un groupe hétéroaryle pentagonal ou hexagonal choisi parmi le groupe constitué des groupes imidazolyle, pyrazolyte, pyrrolyle, triazolyle, tétrazolyle, thiazolyle et oxazolyle,
ou
R1, R2, R3 et R4 représentent indépendamment l'un de l'autre CONR11 R12 ou NR11R12,
R11 et R12 représentent indépendamment l'un de l'autre H, CₑH₂ₑ₊₁, CᵣᵣH₂ᵣᵣ₋₁,
e vaut 1, 2, 3 ou 4,
rr vaut 3, 4, 5 ou 6,
un ou plusieurs atomes de H pouvant être remplacés par des atomes de F dans les groupes CₑH₂ₑ₊₁ et CᵣᵣH₂ᵣᵣ₋₁,
ou
R11 et R12 représentent indépendamment l'un de l'autre un groupe hydroxyéthyle, N,N-diméthylaminoéthyle, N,N-diéthylaminoéthyle, pyrrolidinoéthyle, N-méthylpipérazinoéthyle, pipérazinoéthyle, morpholinoéthyle ou pipéridinoéthyle,
ou
R11 et R12 forment conjointement avec l'atome de N auquel ils sont liés un noyau de pyrrolidine, de pipéridine, de N-méthylpipérazine, de pipérazine ou de morpholine,
ou
R11 et R12 représentent indépendamment l'un de l'autre COR14, CSR14, CONHR14, CSNHR14 ou SO₂R14,
R14 représente C_{g}H_{2g+1},
g vaut 1, 2, 3 ou 4, un ou-plusieurs atomes de H pouvant être remplacés par des atomes de F,
ou
R1, R2, R3 et R4 représentent indépendamment l'un de l'autre OSO₃H, SO₃H, SO₂R₁₅, où
R15 représente CₖH₂ₖ₊₁, OCₗH₂ₗ₊₁ ou NR17R18,
k vaut 1, 2, 3 ou 4, un ou plusieurs atomes de H pouvant être remplacés par des atomes de F,
l vaut 1, 2, 3 ou 4, un ou plusieurs atomes de H pouvant être remplacés par des atomes de F,
R17 et R18 représentent indépendamment l'un de l'autre H, CₘH₂ₘ₊₁ ou CₘH₂ₘ₊₁, où le premier groupe CH₂ fixé sur l'azote est remplacé par CO et le deuxième groupe CH₂ est remplacé par NR19,
m vaut 1, 2, 3, 4 ou 5, un ou plusieurs atomes de H pouvant être remplacés par des atomes de F dans le groupe CₘH₂ₘ₊₁,
R19 représente H ou CₙH₂ₙ₊₁,
n vaut 1, 2, 3 ou 4,
un ou plusieurs atomes de H pouvant être remplacés par des atomes de F dans CₙH₂ₙ₊₁,
ou
R17 et R18 forment conjointement avec l'atome de N, auquel ils sont liés, un noyau pentagonal ou hexagonal,
R5 et R6 représentent indépendamment l'un de l'autre CₚH₂ₚ₊₁,
p vaut 1, 2, 3 ou 4, un ou plusieurs atomes de H pouvant être remplacés par des atomes de F dans CₚH₂ₚ₊₁,
R7 représente H, CₛH₂ₛ₊₁, OCₜH₂ₜ₊₁ ou OCOR22,
s et t valent indépendamment l'un de l'autre 1, 2, 3 ou 4,
un ou plusieurs atomes de H pouvant être remplacés par des atomes de F dans CₛH₂ₛ₊₁ et OCₜH₂ₜ₊₁,
R22 représente CᵤH₂ᵤ₊₁,
u vaut 1, 2, 3 ou 4,
un ou plusieurs atomes de H pouvant être remplacés par des atomes de F dans CᵤH₂ᵤ₊₁,
R8, R9 et R10 représentent indépendamment l'un de l'autre OSO₃H, SO₃H ou SO₂R₂₃,
R23 représente CₙₙH₂ₙₙ₊₁, CₘₘH₂mm-1, OCₚₚH₂ₚₚ₊₁ ou NR25R26,
nn et pp valent indépendamment l'un de l'autre 1, 2, 3, 4 ou 5,
mm vaut 3, 4, 5 ou 6,
un ou plusieurs atomes de H pouvant être remplacés par des atomes de F dans CₙₙH₂ₙₙ₊₁, CₘₘH₂ₘₘ₋₁ et OCₚₚH₂ₚₚ₊₁,
R25 et R26 représentent indépendamment l'un de l'autre H, CN, C_{z}H_{2z+1} ou C_{z}H_{2z+1}, où le premier groupe CH₂ fixé sur l'azote est remplacé par CO ou CS et le deuxième par NR27,
z vaut 1, 2, 3, 4, 5 ou 6, un ou plusieurs atomes de H pouvant être remplacés par des atomes de F dans C₂H_{2z+1},
R27 représente H ou CₐₐH₂ₐₐ₊₁,
aa vaut 1, 2, 3 ou 4,
un ou plusieurs atomes de H pouvant être remplacés par des atomes de F dans CₐₐH₂ₐₐ₊₁,
ou
R25 et R26 forment conjointement avec l'atome de N auquel ils sont liés un noyau pentagonal ou hexagonal,
ou
R27 et un groupe CH₂ de R25 ou R26 forment conjointement avec l'atome de N auquel ils sont liés un noyau pentagonal ou hexagonal,
ou
R8, R9 et R10 représentent indépendamment l'un de l'autre NR32COR30, NR32CSR30 ou NR32SO₂R30,
R30 représente H, OH, C_{cc}H_{2cc+1}, C_{yy}H_{2yy-1}, pyrrolidinyle ou pipéridinyle, noyaux dans lesquels un groupe CH₂ peut être remplacé par O ou NR33,
R32 et R33 représentent indépendamment l'un de l'autre H ou CₕH₂ₕ₊₁,
cc vaut 1, 2, 3, 4, 5 ou 6,
yy vaut 3, 4, 5 ou 6,
h vaut 1, 2, 3 ou 4,
un ou plusieurs atomes de H pouvant être remplacés par des atomes de F et, dans les groupes C_{cc}H_{2cc+1} et C_{yy}H_{2yy-1}, un ou plusieurs atomes de H pouvant être remplacés par des atomes de F et un ou plusieurs groupes CH₂ pouvant être remplacés par O ou NR31,
R31 représente H, CₖₖH₂ₖₖ₊₁, COR65,
kk vaut 1, 2, 3 ou 4, un ou plusieurs atomes de H pouvant être remplacés par des atomes de F,
R65 représente H, CₓₓH₂ₓₓ₊₁,
xx vaut 1, 2, 3 ou 4, un ou plusieurs atomes de H pouvant être remplacés par des atomes de F,
ou
R31 forme conjointement avec un groupe CH₂ de R30 et l'atome de N auquel ils sont conjointement liés un noyau pentagonal ou hexagonal,
ou
R30 représente une substance hétéroaromatique pentagonale ou hexagonale choisie parmi le groupe constitué des groupes pyridyle, imidazolyle, pyrazolyle, pyrrolyle, triazolyle, tétrazolyle, thiényle, thiazolyle et oxazolyle,
qui est non substitué ou substitué par jusqu'à trois substituants choisis parmi le groupe constitué de F, Cl, Br, I, CₒₒH₂ₒₒ₊₁,NR70R71,
R70 et R71 représentent indépendamment l'un de l'autre H, CᵤᵤH₂ᵤᵤ₊₁ et COR72,
R72 représente H, CᵥᵥH₂ᵥᵥ₊₁, oo, uu et w valent indépendamment l'un de l'autre 1, 2, 3 ou 4, un ou plusieurs atomes de H pouvant être remplacés par des atomes de F dans les groupes CₒₒH₂ₒₒ₊₁, CᵤᵤH₂ᵤᵤ₊₁ ou CᵥᵥH₂ᵥᵥ₊₁,
ou
R8, R9 et R10 représentent indépendamment l'un de l'autre H, F, CI, Br, 1, NO_{2,} CN, OH, NH₂, CₑₑH₂ₑₑ₊₁, C_{ww}H_{2ww-1,} OC_{ff}H_{2ff+1} NR40R41, CONR40R41, COOR42, COR42 ou OCOR42,
ee et ff valent indépendamment l'un de l'autre 1, 2, 3 ou 4,
ww vaut 3, 4, 5 ou 6,
un ou plusieurs atomes de H pouvant être remplacés par des atomes de F dans les groupes CₑₑH₂ₑₑ₊₁. C_{ww}H_{2ww-1} et OC_{ff}H_{2ff+1},
R40 et R41 représentent H ou CₜₜH_{2tt+1,}
tt vaut 1, 2, 3, 4, 5, 6, 7 ou 8,
un ou plusieurs atomes de H pouvant être remplacés par des atomes de F dans le groupe CₜₜH₂tt+₁,
ou
R40 et R41 sont à choisir indépendamment l'un de l'autre parmi les groupes hydroxyéthyle, N,N-diméthylaminoéthyle, N,N-diéthylaminoéthyle, pyrrolidinoéthyle, N-méthylpipérazinoéthyle, pipérazinoéthyle, morpholinoéthyle ou pipéridinoéthyle,
ou
R40 et R41 forment conjointement avec l'atome N auquel ils sont liés un noyau choisi parmi le groupe constitué de la pyrrolidine, de la pipéridine, de la N-méthylpipérazine, de la piperazine et de la morpholine,
R42 représente H ou CₕₕH₂ₕₕ₊₁,
hh vaut 1, 2, 3 ou 4,
un ou plusieurs atomes de H pouvant être remplacés par des atomes de F dans le groupe CₕₕH₂ₕₕ₊₁,
Y représente du fluor, du chlore, du brome, un hydroxy, ainsi que toutes les formes anioniques d'acides monocarboxyliques, dicarboxyliques ou tricarboxyliques ou sulfoniques pharmacologiquement compatibles,
ainsi que leurs sels pharmaceutiquement compatibles et trifluoroacétates.

3. Composés de la formule I suivant les revendications 1 et 2, dans lesquels
R1, R2, R3 et R4 représentent indépendamment l'un de l'autre H, F, Cl, Br, OH, NH₂, CₐH₂ₐ₊₁, un groupe cycloalkyle comportant 3, 4, 5 ou 6 atomes de C, OC_{b}H_{2b+1},
a et b valent dans les groupes CₐH₂ₐ₊₁ et OC_{b}H_{2b+1}, indépendamment l'un de l'autre, 1, 2, 3 ou 4, un ou plusieurs atomes de H pouvant être remplacés par des atomes de F,
ou
R1, R2, R3 et R4 représentent indépendamment l'un de l'autre NR11 R12,
R11 et R12 représentent indépendamment l'un de l'autre H, CₑH₂ₑ₊₁, CᵣᵣH₂ᵣᵣ₋₁,
e vaut 1, 2, 3 ou 4,
rr vaut 3, 4, 5 ou 6,
un ou plusieurs atomes de H pouvant être remplacés par des atomes de F dans les groupes CₑH₂ₑ₊₁ et CᵣᵣH₂ᵣᵣ₋₁,
ou
R11 et R12 forment conjointement avec l'atome de N auquel ils sont liés un noyau choisi parmi le groupe constitué de la pyrrolidine, de la pipéridine, de la N-méthylpipérazine, de la pipérazine et de la morpholine,
ou
R11 et R12 représentent indépendamment l'un de l'autre COR14, CSR14, CONHR14, CSNHR14 ou SO₂R14,
R14 représente C_{g}H_{2g+1},
g vaut 1, 2, 3 ou 4, un ou plusieurs atomes de H pouvant être remplacés par des atomes de F,
ou
R1, R2, R3 et R4 représentent indépendamment l'un de l'autre OSO₃H, SO₃H, SO₂R15
R15 représente CₖH₂ₖ₊₁ ou NR17R18,
k vaut 1, 2, 3 ou 4, un ou plusieurs atomes de H pouvant être remplacés par des atomes de F, R17 et R18
représentent indépendamment l'un de l'autre H ou CₘH₂ₘ₊₁,
m vaut 1, 2, 3, 4 ou 5, un ou plusieurs atomes de H pouvant être remplacés par des atomes de F dans le groupe CₘH₂ₘ₊₁,
ou
R17 et R18 forment conjointement avec l'atome de N, auquel ils sont liés, un noyau pentagonal ou hexagonal,
R5 et R6 représentent indépendamment l'un de l'autre un groupe méthyle ou trifluorométhyle, et
R7 représente H,
R8, R9 et R10 représentent indépendamment l'un de l'autre OSO₃H, SO₃H ou SO₂R23,
R23 représente CₙₙH₂ₙₙ₊₁ ou NR25R26,
nn vaut 1, 2, 3, 4 ou 5,
un ou plusieurs atomes de H pouvant être remplacés par de atomes de F dans le groupe CₙₙH₂ₙₙ₊₁,
R25 et R26 représentent indépendamment l'un de l'autre H, CN ou C_{z}H_{2z+1} ou C_{z}H_{2z+1}, où le premier groupe CH₂ fixé sur l'azote est remplacé par CO ou CS et le deuxième groupe CH₂ par NR27,
z vaut 1, 2, 3, 4, 5 ou 6, un ou plusieurs atomes de H pouvant être remplacés par des atomes de F dans C_{z}H_{2z+1},
R27 représente H ou CₐₐH₂ₐₐ₊₁,
aa vaut 1, 2, 3 ou 4, un ou plusieurs atomes de H pouvant être remplacés par des atomes de F dans CₐₐH₂ₐₐ₊₁ ,
ou
R25 et R26 forment conjointement avec l'atome de N auquel ils sont liés un noyau pentagonal ou hexagonal,
ou
R27 et un groupe CH₂ de R25 ou R26 forment conjointement avec l'atome de N auquel ils sont liés un noyau pentagonal ou hexagonal,
ou
R8, R9 et R10 représentent indépendamment l'un de l'autre NR32COR30, NR32CSR30 ou NR32SO₂R30,
R30 représente H, OH, C_{cc}H_{2cc+1,} C_{yy}H_{2yy-1}, ou un groupe pyrrolidinyle ou pipéridinyle, noyaux dans lesquels un groupe CH₂ peut être remplacé par O ou NR33,
R32 et R33 représentent H, du méthyle ou CF₃,
cc vaut 1, 2, 3, 4, 5 ou 6,
yy vaut 3, 4, 5 ou 6,
un ou plusieurs atomes de H pouvant être remplacés par des atomes de F et un ou plusieurs groupes CH₂ pouvant être remplacés par O ou NR31 dans les groupes C_{cc}H_{2cc+1} et C_{yy}H_{2yy-1},
R31 représente H ou un groupe méthyle, éthyle, CF₃, CH₂CF₃, acétyle ou propionyle,
ou
R31 forme conjointement avec un groupe CH₂ de R30 et l'atome de N, auquel ils sont liés conjointement, un noyau pentagonal ou hexagonal,
ou
R30 représente un groupe pyridyle, imidazolyle, pyrazolyle, triazolyle, tétrazolyle, thiazolyle ou oxazolyle qui est non substitué ou substitué par au maximum trois substituants choisis parmi le groupe constitué de F, CI, méthyle, éthyle, trifluorométhyle, NH₂, NHacétyle,
ou
R8, R9 et R10 représentent indépendamment l'un de l'autre H, F, Cl, OH, NH₂, CₑₑH₂ₑₑ₊₁, C_{ww}H_{2ww-1}, OC_{ff}H_{2ff+1}, NR40R41, CONR40R41, COOR42 ou OCOR42,
ee et ff valent indépendamment l'un de l'autre 1, 2, 3 ou 4,
ww vaut 3, 4, 5 ou 6,
un ou plusieurs atomes de H pouvant être remplacés par des atomes de F dans les groupes CₑₑH₂ₑₑ₊₁, C_{ww}H_{2ww-1} et OC_{ff}H_{2ff+1},
R40 et R41 représentent H ou CₜₜH₂ₜₜ₊₁,
tt vaut 1, 2, 3 ou 4,
un ou plusieurs atomes de H pouvant être remplacés par des atomes de F dans le groupe CₜₜH₂ₜₜ₊₁,
ou
R40 et R41 représentent indépendamment l'un de l'autre un groupe hydroxyéthyle, N,N-diméthylaminoéthyle, N,N-diéthylaminoéthyle, pyrrolidinoéthyle, N-méthylpipérazinoéthyle, pipérazinoéthyle, morpholinoéthyle ou pipéridinoéthyle,
ou
R40 et R41 forment conjointement avec l'atome de N auquel ils sont liés un noyau de pyrrolidine, de pipéridine, de N-méthylpipérazine, de pipérazine ou de morpholine,
R42 représente H ou CₕₕH₂ₕₕ₊₁,
hh vaut 1, 2, 3 ou 4,
un ou plusieurs atomes de H pouvant être remplacés par des atomes de F dans le groupe CₕₕH₂ₕₕ₊₁,
Y représente du fluor, du chlore, du brome, un hydroxy, ainsi que toutes les formes anioniques d'acides monocarboxyliques, dicarboxyliques ou tricarboxyliques ou sulfoniques pharmacologiquement compatibles,
ainsi que leurs sels pharmaceutiquement compatibles et trifluoroacétates.

4. Composés de la formule 1 suivant les revendications 1 à 3, dans lesquels
R1 et R3 représentent H,
R2 et R4 représentent indépendamment l'un de l'autre H, F, CI, Br, OH, NH₂, CₐH₂ₐ₊₁, un groupe cycloalkyle comportant 3, 4, 5 ou 6 atomes de C, OC_{b}H_{2b+1},
a et b valent dans les groupes CₐH₂ₐ₊₁ et OC_{b}H_{2b+1}, indépendamment l'un de l'autre, 1, 2, 3 ou 4, un ou plusieurs atomes de H pouvant être remplacés par des atomes de F,
ou
R2 et R4 représentent indépendamment l'un de l'autre NR11R12,
R11 et R12 représentent indépendamment l'un de l'autre H, CₑH₂ₑ₊₁,
e vaut 1, 2, 3 ou 4, un ou plusieurs atomes de H pouvant être remplacés par des atomes de F dans CₑH₂ₑ₊₁,
ou
R11 et R12 forment conjointement avec l'atome de N auquel ils sont liés un noyau choisi parmi le groupe constitué de la pyrrolidine, de la pipéridine, de la N-méthylpipérazine, de la pipérazine et de la morpholine,
ou
R11 et R12 représentent indépendamment l'un de l'autre COR14, CSR14, CONHR14, CSNHR14 ou SO₂R14,
R14 représente C_{g}H_{2g+1},
g vaut 1, 2, 3 ou 4, un ou plusieurs atomes de H pouvant être remplacés par des atomes de F,
ou
R2 et R4 représentent indépendamment l'un de l'autre OSO₃H, SO₃H, SO₂R15.
R15 représente CₖH₂ₖ₊₁ ou NR17R18,
k vaut 1, 2, 3 ou 4, un ou plusieurs atomes de H pouvant être remplacés par des atomes de F,
R17 et R18 représentent indépendamment l'un de l'autre H ou CmH2m+1,
m vaut 1, 2, 3, 4 ou 5, un ou plusieurs atomes de H pouvant être remplacés par des atomes de F dans le groupe CₘH₂ₘ₊₁,
ou
R17 et R18 forment conjointement avec l'atome de N, auquel ils sont liés, un noyau pentagonal ou hexagonal,
R5 et R6 représentent indépendamment l'un de l'autre un groupe méthyle ou trifluorométhyle,
R7 représente H,
R8, R9 et R10 représentent, indépendamment l'un de l'autre, OSO₃H, SO₃H ou SO₂R23,
R23 représente CₙₙH₂ₙₙ₊₁ ou NR25R26,
nn vaut 1, 2, 2, 3, 4 ou 5,
un ou plusieurs atomes de H pouvant être remplacés par des atomes de F dans CₙₙH₂ₙₙ₊₁,
R25 et R26 représentent indépendamment l'un de l'autre H, CN ou C_{z}H_{2z+1} ou un groupe C_{z}H_{2z}+₁ dans lequel le premier groupe CH₂ fixé sur l'azote est remplacé par CO ou CS et le deuxième groupe CH₂ par NR27,
z vaut 1, 2, 3, 4, 5 ou 6, un ou plusieurs atomes de H pouvant être remplacés par des atomes de F dans C_{z}H_{2z+1},
R27 représente H ou CₐₐH₂ₐₐ₊₁,
aa vaut 1, 2, 3 ou 4, un ou plusieurs atomes de H pouvant être remplacés par des atomes de F dans CₐₐH₂ₐₐ₊₁,
ou
R25 et R26 forment conjointement avec l'atome de N auquel ils sont liés un noyau pentagonal ou hexagonal,
ou
R27 et un groupe CH₂ de R25 ou de R26 forment conjointement avec l'atome de N auquel ils sont liés un noyau pentagonal ou hexagonal,
ou
R8, R9 et R10 représentent indépendamment l'un de l'autre NR32COR30, NR32CSR30 ou NR32SO₂R30,
R30 représente H, OH, C_{cc}H_{2cc+1}, C_{yy}H_{2yy-1}, un groupe pyrrolidinyle ou pipéridinyle, noyaux dans lesquels un groupe CH₂ peut être remplacé par O ou NR33,
R32 et R33 représentent H, un groupe méthyle ou CF₃,
cc vaut 1, 2, 3, 4, 5 ou 6,
yy vaut 3, 4, 5 ou 6,
un ou plusieurs atomes de H pouvant être remplacés par des atomes de F et un ou plusieurs groupes CH₂ pouvant être remplacés par O ou NR31 dans les groupes C_{cc}H_{2cc+1} et C_{yy}H_{2yy-1},
R31 représente H ou un groupe méthyle, éthyle, CF₃, CH₂CF₃, acétyle ou propionyle,
ou
R31 forme conjointement avec un groupe CH₂ de R30 et l'atome de N auquel ils sont liés conjointement un noyau pentagonal ou hexagonal,
ou
R30 représente un groupe pyridyle, imidazolyle, pyrazolyle, triazolyle, tétrazolyle, thiazolyle ou oxazolyle qui est non substitué ou substitué par au maximum trois substituants choisis parmi le groupe constitué de F, CI, méthyle, éthyle, trifluorométhyle, NH₂, NHacétyle,
ou
R8, R9 et R10 représentent indépendamment l'un de l'autre H, F, Cl, OH, NH₂, CₑₑH₂ₑₑ₊₁, C_{ww}H_{2ww-1}, OC_{ff}H_{2ff+1}, NR40R41, CONR40R41, COOR42 ou OCOR42,
ee et ff valent indépendamment l'un de l'autre 1, 2, 3 ou 4,
ww vaut 3, 4, 5 ou 6,
un ou plusieurs atomes de H pouvant être remplacés par des atomes de F dans les groupes CₑₑH₂ₑₑ₊₁, C_{ww}H_{2ww-1} et OC_{ff}H_{2ff+1}
R40 et R41 représentent H ou CₜₜH₂ₜₜ₊₁,
tt vaut 1, 2, 3 ou 4,
un ou plusieurs atomes de H pouvant être remplacés par des atomes de F dans le groupe CₜₜH₂ₜₜ₊₁,
ou
R40 et R41 représentent indépendamment l'un de l'autre un groupe hydroxyéthyle, N,N-diméthylaminoéthyle, N,N-diéthylaminoéthyle, pyrrolidinoéthyle, N-méthylpipérazinoéthyle, pipérazinoéthyle, morpholinoéthyle ou pipéridinoéthyle,
ou
R40 et R41 forment conjointement avec l'atome de N auquel ils sont liés un noyau de pyrrolidine, de pipéridine, de N-méthylpipérazine, de pipérazine ou de morpholine,
R42 représente H ou CₕₕH₂ₕₕ₊₁,
hh vaut 1, 2, 3 ou 4,
un ou plusieurs atomes de H pouvant être remplacés par des atomes de F dans le groupe CₕₕH₂ₕₕ₊₁ ,
Y représente du fluor, du chlore, du brome, un hydroxy, ainsi que toutes les formes anioniques d'acides monocarboxyliques, dicarboxyliques ou tricarboxyliques ou sulfoniques pharmacologiquement compatibles,
ainsi que leurs sels pharmaceutiquement compatibles et trifluoroacétates.

5. Composé suivant l'une des revendications 1 à 4, **caractérisé en ce qu'**il est
a. du trifluoroacétate de 6,8-dichloro-2,2-diméthyl-4-phényl-1,2,3,4-tétrahydro-isoquinoléinium,
b. du trifluoroacétate de 6,8-dichloro-2,2-diméthyl-4-(4-sulfamoyl-phényl)-1,2,3,4-tétrahydro-isoquinoléinium,
c. de l'iodure de 4-(4-acétylamino-phényl)-6,8-dichloro-2,2-diméthyl-1,2,3,4-tétrahydro-isoquinoléinium,
d. de l'iodure de (+)-4-(4-acétylamino-phényl)-6,8-dichloro-2,2-diméthyl-1 ,2,3,4-tétrahydro-isoquinoléinium,
e. de l'iodure de (-)-4-(4-acétylamino-phényl)-6,8-dichloro-2,2-diméthyl-1,2,3,4-tétrahydro-isoquinoléinium,
f. du chlorure de (+)-4-(4-acétylamino-phényl)-6,8-dichloro-2,2-diméthyl-1,2,3,4-tétrahydro-isoquinoléinium,
g. du chlorhydrate; de chlorure de 4-(4-amino-phényl)-6,8-dichloro-2,2-diméthyl-1,2,3,4-tétrahydro-isoquinoléinium,
h. du chlorure de 6,8-dichloro-4-[4-(3-éthyl-uréido)-phényl]-2,2-diméthyl-1,2,3,4-tétrahydro-isoquinoléinium,
ainsi que leurs sels pharmaceutiquement compatibles.

6. Utilisation d'un composé I suivant la revendication 1, pour la préparation d'un médicament destiné au traitement ou à la prophylaxie de troubles du mouvement respiratoire.

7. Utilisation d'un composé I suivant la revendication 1, pour la préparation d'un médicament destiné au traitement ou à la prophylaxie de troubles de la respiration, en particulier de troubles de la respiration conditionnée pendant le sommeil, comme les apnées du sommeil.

8. Utilisation d'un composé I suivant la revendication 1, pour la préparation d'un médicament destiné au traitement ou à la prophylaxie du ronflement.

9. Utilisation d'un composé I suivant la revendication 1, pour la préparation d'un médicament destiné au traitement ou à la prophylaxie de maladies des reins aiguës et chroniques, en particulier de la défaillance aiguë et de la défaillance chronique des reins.

10. Utilisation d'un composé I suivant la revendication 1, pour la préparation d'un médicament destiné au traitement ou à la prophylaxie de troubles de la fonction intestinale.

11. Utilisation d'un composé I suivant la revendication 1, pour la préparation d'un médicament destiné au traitement ou à la prophylaxie de troubles de la fonction biliaire.

12. Utilisation d'un composé I suivant la revendication 1, pour la préparation d'un médicament destiné au traitement ou à la prophylaxie d'états ischémiques du système nerveux périphérique et central et de l'attaque d'apoplexie.

13. Utilisation d'un composé I suivant la revendication 1, pour la préparation d'un médicament destiné au traitement ou à la prophylaxie d'états ischémiques d'organes périphériques et de membres.

14. Utilisation d'un composé I suivant la revendication 1, pour la préparation d'un médicament destiné au traitement d'états de choc.

15. Utilisation d'un composé I suivant la revendication 1, pour la préparation d'un médicament destiné à la mise en oeuvre lors d'opérations chirurgicales et de transplantations d'organes.

16. Utilisation d'un composé I suivant la revendication 1, pour la conservation et l'entreposage d'éléments transplantés pour mesures chirurgicales.

17. Utilisation d'un composé I suivant la revendication 1, pour la préparation d'un médicament destiné au traitement de maladies au cours desquelles la prolifération cellulaire représente une cause primaire ou secondaire.

18. Utilisation d'un composé I suivant la revendication 1, pour la préparation d'un médicament destiné au traitement ou à la prophylaxie de troubles du métabolisme des graisses.

19. Utilisation d'un composé 1 suivant la revendication 1, pour la préparation d'un médicament destiné au traitement ou à la prophylaxie de l'attaque par des ectoparasites.

20. Remède contenant une quantité efficace d'un composé I suivant la revendication 1.
